(19) Europäisches Patentamt
European Patent Office
Office européen des brevets

(11) EP 0 625 209 B1

(12) FASCICULE DE BREVET EUROPEEN

(45) Date de publication et mention de la délivrance du brevet:
**17.07.1996 Bulletin 1996/29**

(21) Numéro de dépôt: **93901768.7**

(22) Date de dépôt: **25.11.1992**

(51) Int Cl.[6]: **C12Q 1/28**, C07D 311/94, C07C 39/17, C07C 215/74, C07C 229/18, C07C 229/54, C07C 309/46

(86) Numéro de dépôt international:
**PCT/FR92/01093**

(87) Numéro de publication internationale:
**WO 93/11258 (10.06.1993 Gazette 1993/14)**

# (54) PROCEDE DE DOSAGE DE L'ACTIVITE SOD UTILISANT UN COMPOSE AUTOXYDABLE, NECESSAIRE POUR SA MISE EN OEUVRE, COMPOSES AUTOXYDABLES ET LEUR PREPARATION

VERFAHREN ZUR BESTIMMUNG DER SOD-AKTIVITÄT UNTER VERWENDUNG EINER AUTOXYDABLEN ZUSAMMENSETZUNG, DIESBEZÜGLICHER TESTSATZ, AUTOXYDABLE ZUSAMMENSETZUNGEN UND DEREN HERSTELLUNGSVERFAHREN

METHOD FOR ASSAYING THE SOD ACTIVITY BY USING A SELF- OXIDIZABLE COMPOUND NECESSARY FOR ITS IMPLEMENTATION, SELF- OXIDIZABLE COMPOUNDS AND PREPARATION THEREOF

(84) Etats contractants désignés:
**DE FR GB IT**

(30) Priorité: **29.11.1991 FR 9114781**
**24.02.1992 FR 9202082**

(43) Date de publication de la demande:
**23.11.1994 Bulletin 1994/47**

(73) Titulaire: **OXIS INTERNATIONAL S.A.**
**F-94385 Bonneuil sur Marne (FR)**

(72) Inventeurs:
- **XU, Jinzhu**
  **F-94200 Ivry-sur-Seine (FR)**
- **YADAN, Jean-Claude, Yomtob**
  **F-75011 Paris (FR)**
- **MOUTET, Marc, Emmanuel, Georges**
  **F-92220 Bagneux (FR)**
- **CHAUDIERE, Jean, Raymond**
  **F-94100 Saint-Maur-des-Fossés (FR)**

(74) Mandataire: **Portal, Gérard et al**
**Cabinet Beau de Loménie**
**158, rue de l'Université**
**75340 Paris Cédex 07 (FR)**

(56) Documents cités:
**WO-A-86/05689** **GB-A- 2 065 302**
**US-A- 4 569 766**

- **METHODS IN ENZYMOLOGY, vol. 186, 1990; J.P. MARTIN, pp. 220-227**
- **ARCHIV OF BIOCHEMISTRY & BIOPHYSICS; vol. 255, no. 2, juin 1987; J.P. MARTIN et al., pp. 329-336**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne le dosage de l'activité superoxyde dismutase (SOD).

Elle a plus particulièrement pour objet un nouveau procédé de dosage ce l'activité SOD, notamment dans des échantillons biologiques, à l'aide de composés autoxydables définis plus loin, des nécessaires ou kits pour la mise en oeuvre Ce ce procédé et des composés nouveaux utilisables dans ce procédé, ainsi que leur préparation.

Les tissus des organismes aérobies et ceux de l'organisme humain en particulier, sont le siège d'une production continue d'ion superoxyde $O_2 \cdot^-$, qui résulte du processus de respiration cellulaire, ainsi que de nombreuses voies métaboliques essentielles.

Cette production d'ion superoxyde augmente fortement lorsque ces organismes sont soumis à un "stress oxydant" d'origine toxicologique (hyperoxie, irradiation, intoxication par des xénobiotiques générateurs de radicaux libres, notamment) ou physiopathologique (inflammation, ischémie, reperfusion post-ischémique, notamment).

Il est admis par l'ensemble de la communauté scientifique que l'ion superoxyde est très toxique, bien que les mécanismes sous-jacents à cette toxicité restent sujets à controverse (référence 1).

Pour se protéger contre les effets délétères de l'ion superoxyde, les organismes aérobies possèdent des systèmes enzymatiques, les superoxyde dismutases (SOD), qui catalysent la dégradation de l'ion superoxyde selon la réaction de dismutation suivante :

$$2\,O_2 \cdot^- + 2\,H^+ \rightarrow H_2O_2 + O_2$$

Compte tenu du rôle protecteur majeur de cette réaction de dismutation vis-à-vis de la toxicité de l'ion superoxyde, l'activité catalytique des SOD intra- et extra-cellulaires conditionne la survie des tissus d'un organisme aérobie (références 2 à 5).

Le dosage de l'activité SOD présente donc un intérêt majeur pour les chercheurs biologistes comme pour les laboratoires de Chimie Clinique, à condition de pouvoir être réalisé à l'aide d'une méthode sensible, spécifique, rapide et peu complexe.

De nombreuses méthodes de dosage de l'activité SOD ont été publiées et critiquées (références 6 à 10), mais il est généralement admis qu'il n'existe toujours pas de compromis satisfaisant entre fiabilité et complexité des méthodes existantes, car la mesure d'une activité SOD pose deux problèmes majeurs qu'aucune des méthodes publiées à ce jour n'a permis de résoudre de façon simple.

Le premier problème est celui de la décomposition spontanée de l'unique substrat, l'ion superoxyde, qui est très rapide à pH voisin de 7, et qui bien que fortement ralentie, reste significative à pH 9, pH au-delà duquel certains types de SOD naturelles sont rapidement inactivées. L'obtention d'une concentration stationnaire suffisamment stable requiert donc la présence d'une source dynamique d'ion superoxyde dans la solution de dosage.

Les sources électrolytiques requièrent un équipement complexe et une préextraction du catalyseur SOD, ce qui est prohibitif en Chimie Clinique.

Les sources photochimiques telles que la riboflavine conduisent à des méthodes non standardisables et de mise en oeuvre complexe.

Les sources enzymatiques telles que la xanthine oxydase requièrent des solutions à préparer extemporanément. Elles sont chères, source d'artéfacts et difficilement automatisables.

Les sources purement chimiques se réduisent à des composés autoxydables tels que la 6-hydroxydopamine, le pyrogallol, l'hydroxylamine ou l'ion sulfite, dont l'autoxydation est généralement inhibée par la SOD.

Ces réactifs chimiques donnent rarement des résultats précis et sensibles sur extraits biologiques bruts, et ils requièrent la préparation extemporanée d'une solution anaérobie de réactif; leur utilisation est donc difficilement automatisable.

Le second problème tient au fait qu'il est difficile de mesurer directement (dosage "direct") la disparition du substrat superoxyde $O_2 \cdot^-$ en fonction du temps, ou l'apparition de ses produits de dismutation, l'eau oxygénée $H_2O_2$ (peroxyde d'hydrogène) et l'oxygène, compte tenu des concentrations et des pH accessibles dans les conditions d'un dosage d'activité SOD.

La seule méthode spectrophotométrique "directe" est celle de MARKLUND (références 11 et 12) qui mesure la vitesse de disparition de l'ion superoxyde à pH 9,5, ce qui ne permet pas le dosage de toutes les SOD naturelles.

Cette méthode requiert un spectrophotomètre rapide. Les fortes concentrations d'ion superoxyde et la longueur d'onde (250 nm) utilisées rendent la méthode peu précise, ce qui oblige l'expérimentateur à faire plusieurs mesures pour obtenir une valeur moyenne. De plus, il est nécessaire d'ajouter de la catalase pour éviter l'inactivation de la SOD-Cu/Zn, et il faut repréparer une nouvelle solution de superoxyde de potassium immédiatement avant chaque dosage.

Cette méthode spectrophotométrique directe est donc délicate à mettre en oeuvre, et il ne semble pas possible de l'améliorer de façon significative.

Le corollaire est que les méthodes populaires, c'est-à-dire auxquelles on a le plus souvent recours, sont généralement indirectes : elles reposent sur la compétition entre l'activité SOD que l'on cherche à mesurer, et la réaction de l'ion superoxyde avec un piégeur chromogène tel que le cytochrome C ferrique ou le nitrobleu de tétrazolium (NBT), dont le produit réactionnel peut être mesuré par spectrophotométrie UV/visible. Un piégeur chimiluminescent, tel que le luminol ou la lucigénine, peut également être utilisé avec une plus grande sensibilité de la mesure, mais les problèmes d'interférences et de disponibilité instrumentale excluent l'utilisation de telles méthodes par un laboratoire non spécialiste.

Outre la spécificité souvent insuffisante des réactifs chromogènes vis-à-vis de l'ion superoxyde, le principal inconvénient des dosages par compétition est qu'ils requièrent une linéarisation des valeurs d'inhibition (par la SOD), sur 5 ou 6 prises d'échantillons de dilutions croissantes.

Enfin, on connaît un certain nombre d'immunodosages de SOD-Cu/Zn ou de SOD-Mn.

Certains sont très sensibles, mais ils ne renseignent pas sur l'activité enzymatique de l'échantillon qui dépend notamment du degré d'inactivation éventuelle de l'enzyme. Ils sont par ailleurs longs à mettre en oeuvre et relativement complexes.

Artéfacts de mesure et démultiplication du dosage sont donc intrinsèquement liés à toutes les méthodes spectrophotométriques accessibles à la plupart des laboratoires, ce qui ne satisfait pas les besoins des laboratoires de recherche et reste incompatible avec les impératifs de standardisation de la Chimie Clinique.

Si l'on a recours à la spectrophotométrie UV/visible, il semble difficile, voire impossible, de mettre au point une méthode de dosage qui soit à la fois sensible, plus spécifique, plus simple et plus fiable que les méthodes existantes, sans avoir recours au suivi en cinétique d'une réaction activée par l'activité SOD plutôt qu'à celui d'une réaction inhibée par l'activité SOD ou à une méthode "directe".

A cet égard, une méthode récente (références 13 et 14), utilisant un colorant autoxydable, l'hématoxyline, se distingue des autres, dans la mesure où elle exploite une activation de l'autoxydation du réactif par l'activité SOD.

En effet, l'hématoxyline s'autoxyde à pH > 6,5 (référence 13). La vitesse d'autoxydation augmente d'un facteur proche de 100 entre pH 7 et 9. La SOD inhibé cette autoxydation à pH < 8,1, alors qu'elle l'active à pH > 8,1. Cette observation a conduit à la mise au point d'un dosage, basé sur le suivi spectrophotométrique de la formation d'hématéine à 560 nm, qui est accélérée par la SOD à pH alcalin. La mise en oeuvre de cette méthode est délicate du fait de l'instabilité du réactif et elle reste tributaire d'une phase de préparation de l'échantillon (dialyse) pour éliminer les interférences dues à la présence de composés réducteurs, comme par exemple le glutathion.

Par ailleurs, la faible sensibilité du dosage (à pH > 8,1), liée en partie à la décomposition du produit d'oxydation mesuré, l'hématéine, mais également à la présence de deux sites d'oxydation (catéchols) potentielle, limitent l'intérêt et l'exploitation de cette méthode.

En conclusion, la méthode à l'hématoxyline, bien que présentant un certain intérêt, présente des inconvénients rhédibitoires qui ne permettent pas son utilisation générale pour le dosage de l'activité SOD, en particulier dans des échantillons biologiques.

La présente invention a notamment pour buts de :

- disposer d'un réactif dont le produit d'autoxydation soit stable pendant la mesure ;
- augmenter la sensibilité du dosage par rapport à une méthode utilisant l'hématoxyline (dans des conditions d'activation de l'autoxydation) ;
- pouvoir éliminer les interférences éventuelles les plus probables telles que celles liées à la présence de mercaptans comme le glutathion, par exemple ;
- mettre au point un procédé utilisant un milieu réactionnel de pH inférieur ou égal à 9, de façon à permettre un dosage de l'activité des SOD connues, sans risque d'inactivation rapide.

Ces buts sont atteints selon l'invention qui utilise des réactifs chimiques définis ci-après présentant la propriété de s'autoxyder dans une solution oxygénée, pour conduire à un produit chromophorique dont la cinétique d'apparition est augmentée en présence d'un catalyseur du type SOD.

La structure de ces réactifs est caractérisée, en particulier, par un cycle aromatique à fonction catéchol, lié par l'intermédiaire d'un carbone benzylique tertiaire à un cycle aromatique, substitué par un groupement électrodonneur.

Le produit d'autoxydation de ces réactifs présente une longueur d'onde d'absorption très différente de celle caractérisant le réactif lui-même.

L'invention fournit donc un procédé mettant en oeuvre ces réactifs, qui permet de doser l'activité SOD de tout catalyseur en milieu aqueux, à l'aide d'une seule mesure d'absorption UV/visible et donc la simplicité de mise en oeuvre est nettement supérieure à celle des autres méthodes spectrophotométriques préalablement décrites.

L'invention fournit également un procédé de stabilisation d'une solution-mère des réactifs chimiques définis ci-après, au moyen de dérivés du bore, en particulier l'acide borique et ses dérivés tels que l'acide phénylborique.

L'invention fournit en outre un procédé de dosage de l'activité SOD du type décrit ci-dessus, en présence de mercaptans, grâce à l'utilisation d'un réactif rapide des groupements thiols qui permet d'éliminer les interférences

correspondantes, en particulier celles qui sont dues à la présence de glutathion ou d'albumine dans l'échantillon.

Selon l'un de ses aspects, l'invention a pour objet un procédé de dosage de l'activié superoxyde dismutase (SOD) dans un milieu liquide, procédé qui utilise l'activation de l'autoxydation d'un réactif par l'activité SOD, caractérisé en ce que ledit réactif est un composé répondant à la formule générale I :

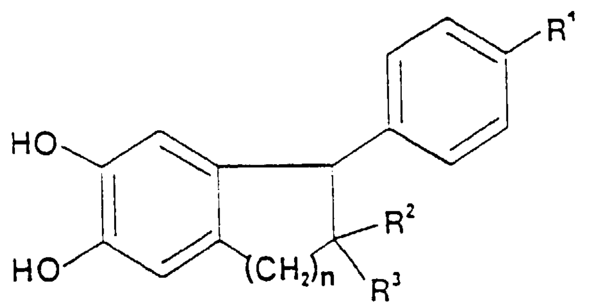

(I)

dans laquelle :

soit :

n = 1 ou 2,
$R^1$ = $-OR^4$ ou $-NR^5R^6$,
$R^2$ = hydrogène, $-OR^4$, alkyle (avec 1 à 6 atomes de carbone), ou bien $-CH_2-$ ou $-CH_2-CH_2-$, pour former un cycle par liaison avec le substituant phényle, en position méta par rapport à $R^1$ et
$R^3$ = hydrogène, alkyle (avec 1 à 6 atomes de carbone) ou $-OR^4$ (à condition que $R^2$ soit différent de $-OR^4$), avec
$R^4$ = hydrogène ou alkyle (avec 1 à 6 atomes de carbone),
$R^5$ = hydrogène, alkyle (avec 1 à 6 atomes de carbone), $-CH_2COOH$, $-C_6H_5COOH$ ou $-C_6H_5SO_3H$ et
$R^6$ = hydrogène, alkyle (avec 1 à 6 atomes de carbone) ou $-CH_2COOH$ ;

soit :

n = 1,
$R^1$ = $-OR^4$ ($R^4$ étant défini comme précédemment),
$R^2$ = $-CH_2-O-$ ,pour former un cycle par liaison de l'atome d'oxygène avec le substituant phényle, en position méta par rapport à $R^1$, et
$R^3$ = hydrogène ou $-OR^4$ ($R^4$ étant défini comme précédemment).
Le composé de formule générale I ci-dessus dans laquelle
n = 1, $R^1 = -OH$, $R^2 = -CH_2-O-$ et $R^3 = -OH$ est la braziline qui est un produit commercial.

Les autres composés répondant à la formule générale I définie ci-dessus peuvent être preparés selon les procédures générales décrites plus loin, généralement à partir de produits commerciaux.

Toutefois, les composés dans lesquels R5 représente $-C_6H_5COOH$ ou $-C_6H_5SO_3H$ sont obtenus à partir au synthon de type parabromophénylaminophényle substitué de façon appropriée. Ce synthon est préparé selon la procédure générale suivante.

Le paranitrophénol est alkylé au moyen d'un ester de l'acide chloroacécique. Le dérivé obtenu est saponifié, puis amidifié avec de la parabromoaniline. L'amide correspondant est soumis à la réaction de Smiles (référence 15) pour conduire au dérivé diphénylaminé correspondant. Ce dernier est réduit, diazoté et substitué de façon appropriée pour conduire au synthon désiré.

**Procédure générale de synthèse des composés de la série I (n = 1 ou 2, $R^1$ = $-OR^4$ ou $-NR^5R^6$, $R^2$ = hydrogène ou alkyle et $R^3$ = hydrogène ou alkyle) :**

La 5,6-dihydroxindan-1-one ou la 6,7-dihydroxy-1-tétralone (préparée à partir du vératrole), convenablement protégée par un groupe alkyle ou aralkyle comme par exemple le groupe méthyle ou benzyle, ou par un groupe de type silyle comme par exemple le groupe terbutyldiméthylsilyle, est traitée soit directement, soit après mono- ou bis-alkylation préalable (de la position en $\alpha$ de la fonction cétone) à l'aide d'un agent alkylant tel que l'iodure de méthyle par exemple, par le réactif organomagnésien correspondant au bromure de phényle substitué en para par un groupement oxygéné, tel que par exemple le groupe méthoxy, ou azoté, tel que par exemple le groupe diméthylamino.

Les intermédiaires, ainsi obtenus, sont réduits puis déprotégés pour conduire aux composés de la série I.

Le schéma réactionnel qui suit illustre cette procédure.

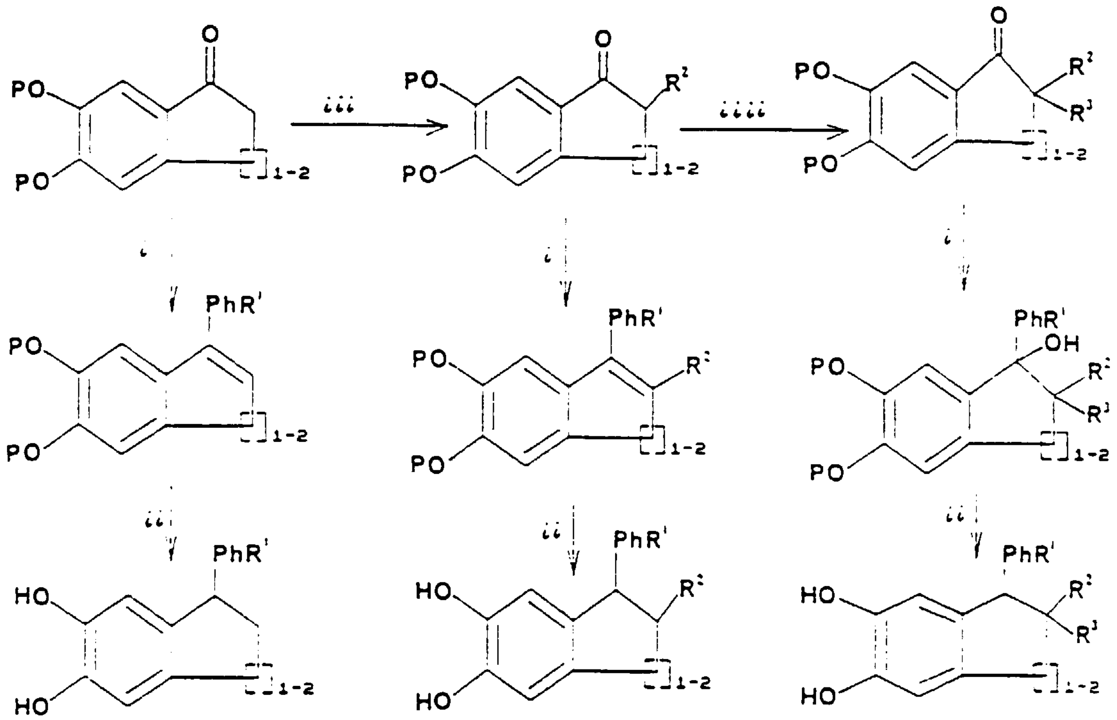

| | |
|---|---|
| *i* : | $R^1PhMgBr$ (R1 en position para) |
| *ii* : | réduction puis déprotection |
| *iii* : | base, $R^2X$ |
| *iiii* : | base, $R^3X$ |
| P : | groupe protecteur |

**Procédure générale de synthèse des composés de la série II (n = 1 ou 2, $R^1$ = $-OR^4$ ou $-NR^5R^6$, $R^2$ = hydrogène ou alkyle et $R^3$ = $-OR^4$).**

Ces composés sont obtenus par hydratation des alcènes, intermédiaires de synthèse des composés de la série I, après hydroboration et oxydation; puis les alcools ainsi obtenus sont éventuellement alkylés avec un halogénure d'alkyle comme par exemple l'iodure de méthyle, et enfin déprotégés.

Le schéma réactionnel qui suit illustre cette procédure.

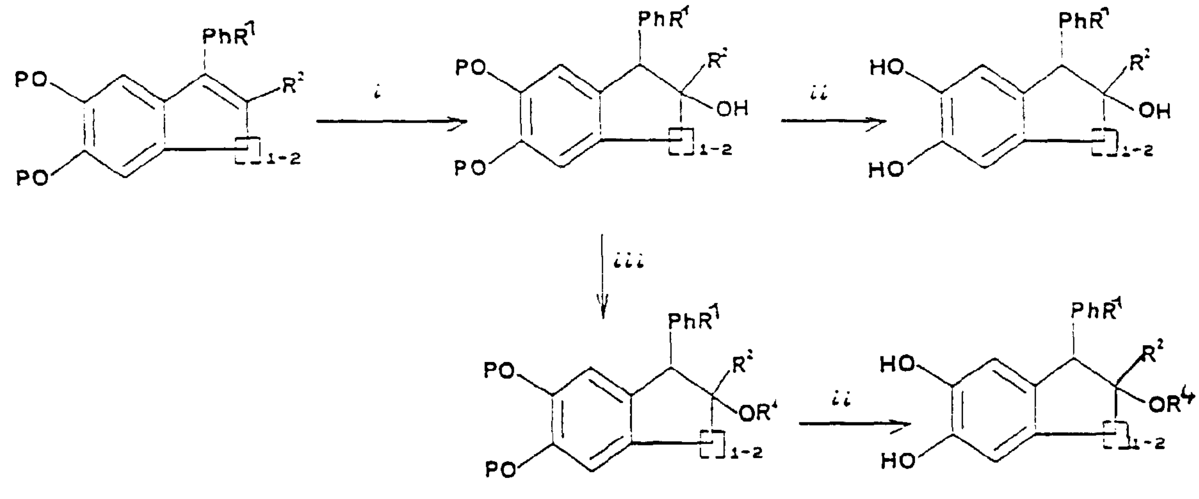


*i* : 1) $BH_3$ : 2) $H_2O_2$, NaOH
*ii* : déprotection
*iii* : base, $R^4X$
P : groupe protecteur

**Procédure générale de synthèse des composés de la série III (n = 1 ou 2, $R^1$ = $-OR^4$ ou $-NR^5R^6$ et $R^2$ = $-CH_2-$ ou $-CH_2-CH_2-$ formant un cycle par liaison avec le substituant phényle, en position méta par rapport à $R^1$) :**

Pour synthétiser ces composés, le produit de crotonisation entre le 3,4-dihydroxybenzaldéhyde, protégé de façon appropriée, et la tétral-1-one ou l'indan-1-one convenablement substituées, est soit directement, soit indirectement (après préparation de l'homologue supérieur par l'intermédiaire d'un dérivé cyclopropanique) réduit par hydrogénation catalytique en dérivé saturé correspondant. Ce dernier est cyclisé en présence d'acide polyphosphorique et l'alcène ainsi obtenu est réduit puis déprotégé pour conduire au composé de la série III recherché.

Le schéma réactionnel qui suit illustre cette procédure.

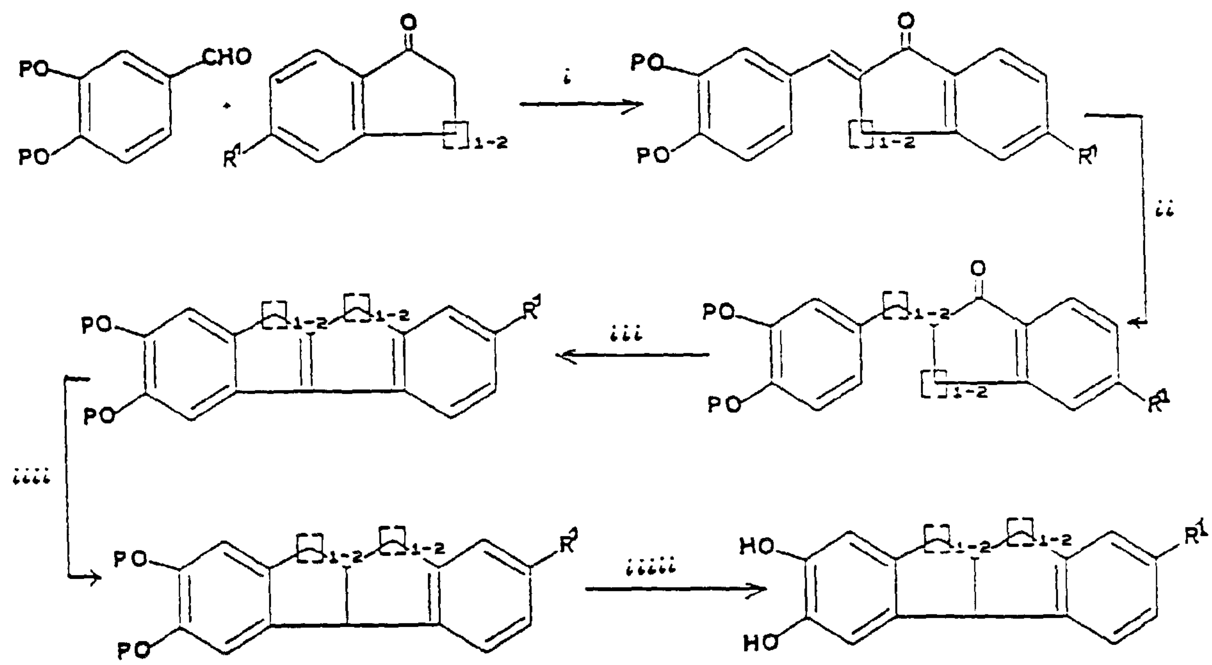


*i* : $H^+$
*ii* : réduction (ou préparation de l'homologue supérieur puis réduction)

*iii* : acide polyphosphorique
*iiii* : réduction
*iiiii* : déprotection
P : groupe protecteur

**Procédure générale de synthèse des composés de la série IV (n = 1, $R^1$ = -OH, $R^2$ = $-CH_2-O-$, pour former un cycle par liaison de l'atome d'oxygène avec le substituant phényle, en position méta par rapport à $R^1$ et $R^3$ = hydrogène ou $-OR^4$ ) :**

Ces composés peuvent être synthétisés à partir de la braziline, selon le schéma réactionnel suivant :

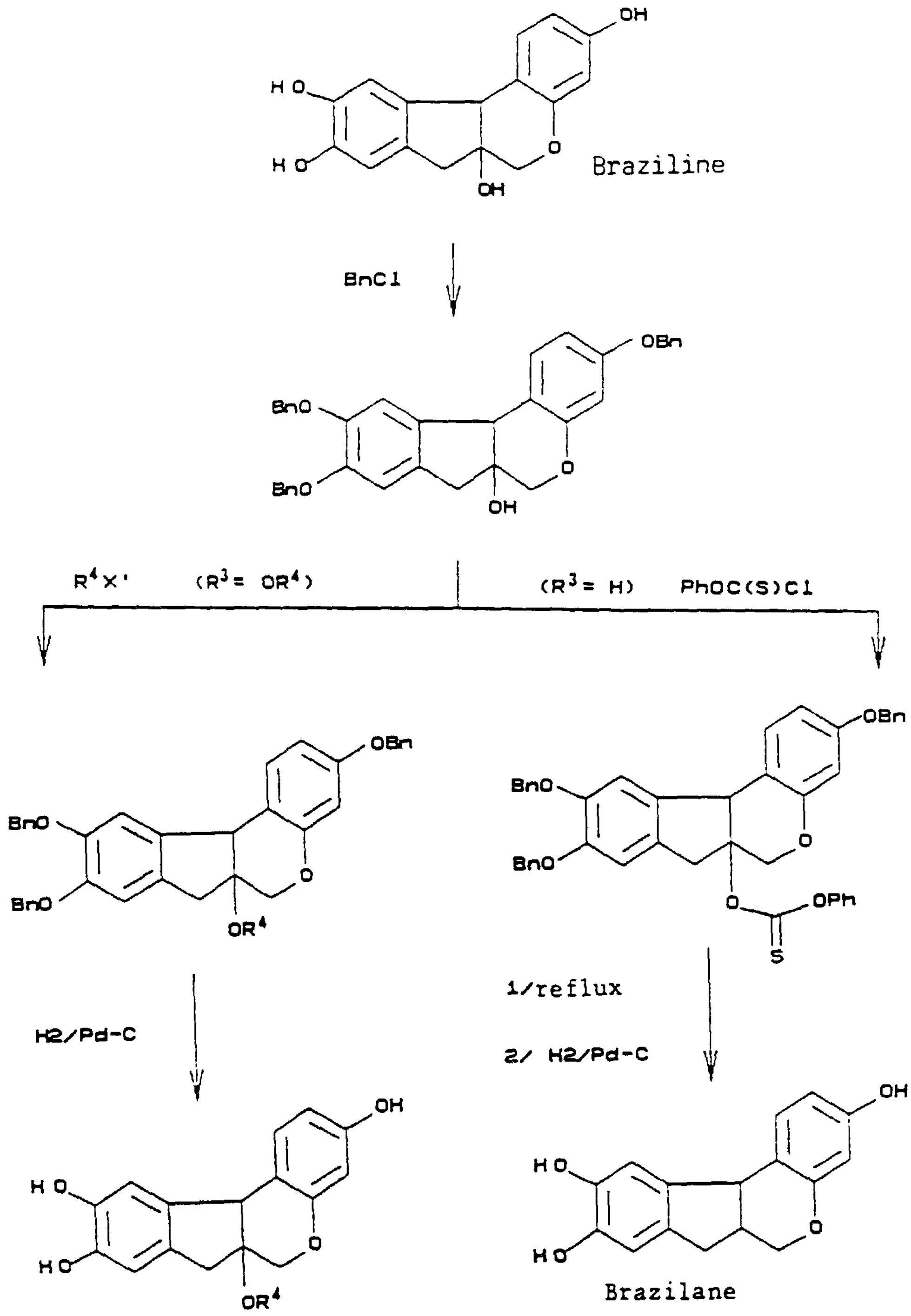


Dans ce schéma :

- Bn représente un groupe protecteur ces OH phénoliques tel que le groupe benzyle,
- $R^4$ X' représente un agent alkylant.

Après avoir protégé les 3 hydroxyles phénoliques sous forme par exemple d'éther benzylique, le groupement alcool tertiaire est éliminé sous forme de phénylthionocarbonate, par exemple ou bien alkylé à l'aide, par exemple, d'iodure de méthyle. Le produit désiré est obtenu après déprotection par hydrogénation catalytique, notamment en présence de palladium sur charbon, concomittante une réduction dans le cas du brazilane.

Les exemples 9 et 10 de la partie expérimentale qui suit illustrent la préparation de composés de ce type.

**Procédure générale de synthèse des composés de la série V (n = 1, $R^1$ = -O-alkyle, $R^2$ = $-CH_2-O-$, pour former un cycle par liaison de l'atome d'oxygène avec le substituant phényle, en position méta par rapport à $R^1$ et $R^3$ = hydrogène ou $-OR^4$ ) :**

La braziline ou un de ses dérivés, dans lequel $R^1$ = OH et $R^3$ est différent de OH, est protégé sur ses deux hydroxyles catécholiques par un pont méthylène, par exemple au moyen de formaldéhyde. Le dérivé obtenu est alkylé sur son hydroxyle non protégé, au moyen d'un agent alkylant tel que par exemple un halogénure d'alkyle, puis déprotégé en milieu acide.

L'exemple 11 de la partie expérimentale qui suit illustre la préparation d'un composé de ce type dans lequel $R^1$ = -O-méthyle et $R^3$ = $-OR^4$, avec $R^4$ = hydrogène.

Tous les dérivés de la braziline des séries IV et V sont obtenus par modification chimique de ses groupements hydroxyles. Les modification apportées permettent de moduler la sensibilité du dosage de l'activité SOD.

Lorsque le dosage de l'activité SOD doit être réalisé dans un milieu renfermant un ou plusieurs composés comprenant un ou plusieurs groupements thiol, tel que notamment un milieu biologique, le pouvoir réducteur de ces derniers est susceptible de provoquer des interférences qu'il convient d'éliminer.

De telles interférences peuvent être facilement évitées en ajoutant au milieu dans lequel le dosage est effectué au moins un réactif choisi parmi les composés de types pyridinium et quinoléinium dont l'utilisation en tant que "piégeurs de mercaptans" fait l'objet de la demande de brevet français déposée le 29 novembre 1991 au nom de BIOXYTECH et publié sous le n° 2684375 et qui à pour titre : "Réactifs piégeurs de mercaptans, leur préparation et leurs applications".

Ces réactifs répondent aux critères suivants :

- spécificité vis-à-vis des mercaptans dans les conditions opératoires utilisées,
- rapidité de réaction,
- non-interférence avec les autres réactifs utilisés dans le dosage de l'activité SOD.

Plus précisément, selon un autre de ses aspects, l'invention a pour objet un procédé de dosage de l'activité superoxyde dismutase (SOD) dans un milieu liquide contenant un ou plusieurs mercaptan(s), notamment un milieu biologique, procédé qui utilise l'activation de l'autoxydation d'un réactif par l'activité SOD, caractérisé en ce que ledit réactif est un composé répondant à la formule générale I définie ci-dessus et en ce que le milieu contient en outre une quantité, capable de piéger totalement lesdits mercaptans par S-alkylation, d'au moins un composé répondant à la formule générale II :

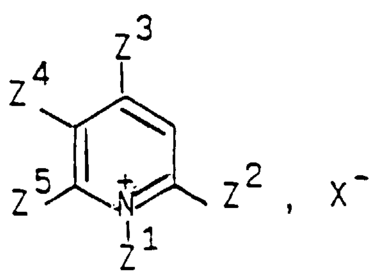

(II)

dans laquelle :

- $Z^1$ = alkyle (avec 1 à 6 atomes de carbone), benzyle, p-nitrobenzyle, phényle, o,p-dinitrophényle, $-CH_2-COOH$ ou $-CH_2-CH_2-COOH$;
- $Z^4$ = hydrogène et
- $Z^5$ = hydrogène ou alkyle (avec 1 à 6 atomes de carbone),

ou

$Z^4$ et $Z^5$ forment ensemble, avec les deux atomes ce carbone intermédiaires, un cycle phényle;
X = halogénure, sulfonate, fluorosulfonate, phosphonate, tétrafluoroborate ou tosylate; et

soit

$Z^2$ = vinyle, et
$Z^3$ = hydrogène ou alkyle (avec 1 à 6 atomes de carbone) (composés 2-vinyliques),

soit

$Z^3$ = vinyle, et
$Z^2$ = hydrogène ou alkyle (avec 1 à 6 atomes de carbone) (composés 4-vinyliques).

On entend par "sulfonate" un anion de formule générale $R\text{-}SO_3^-$ dans laquelle R représente, par exemple, trifluorométhyle.

Ces composés sont en partie connus ou peuvent être synthétisés par analogie à des procédés utilisés pour la synthèse de composés connus.

Ainsi les composés 2-vinyliques peuvent être synthétisés comme suit.

Le dérivé initial (pyridine ou quinoléine substituée) est soumis à la méthode de Comins (référence 16), à l'aide de bromure de vinylmagnésium. Le composé ainsi obtenu est oxydé avec, par exemple, de la tétrachloro-1,4-benzoquinone (parachloranil) ou du soufre $S_8$. L'alkylation de l'azote hétérocyclique est réalisée à l'aide d'un agent alkylant, par exemple le tétrafluoroborate de triméthyloxonium, le sulfate de méthyle, le bromure de benzyle, le fluorodinitrobenzène, l'iodoacétate de sodium ou le 3-bromopyruvate ce sodium, choisi selon la signification de $Z^1$.

L'exemple 12 de la partie expérimentale qui suit illustre la préparation d'un composé de ce type.

Par ailleurs, les composés 4-vinyliques peuvent être synthétisés comme suit.

L'aldéhyde hétérocyclique correspondant (fonction aldéhyde, -CHO, en position 4) est soumis à la réaction de Wittig, avec, par exemple, le bromure de triphénylméthylphosphonium, ou à la réaction de Peterson, avec par exemple, le chlorométhyltriméthylsilane. L'alkylation de l'azote hétérocyclique est réalisée à l'aide d'un agent alkylant, par exemple le tétrafluoroborate de triméthyloxonium, le sulfate de méthyle, le bromure de benzyle, le fluorodinitrobenzène, l'iodoacétate de sodium ou le 4-bromopyruvate de sodium, choisi selon la signification de $Z^1$.

L'exemple 13 de la partie expérimentale qui suit illustre la préparation d'un composé de ce type.

Le procédé selon l'invention, notamment lorsqu'il met en oeuvre au moins un "piégeur de mercaptans" défini ci-dessus, permet de doser l'activité SOD de tout catalyseur en milieu aqueux, à l'aide d'une seule mesure d'absorption UV/visible dont la simplicité de mise en oeuvre est nettement supérieure à celle des méthodes spectrophotométriques préalablement décrites.

Ce nouveau procédé de dosage constitue donc un outil de choix pour les recherches biologiques en général ainsi qu'en Chimie Clinique, en particulier pour l'élaboration de nécessaires ou kits de dosage.

Dans ce dernier cas, ce dosage est susceptible d'apporter une information médicalement utile dans des états physiopathologiques tels que les maladies d'origine inflammatoire ou ischémique, les états de choc, les états infectieux, les maladies systémiques et les maladies auto-immunes, les tumeurs, les états de malnutrition, les maladies associées au vieillissement, les affections dégénératives, les anémies hémolytiques et les syndrômes d'intoxication par des polluants de l'environnement ou par des médicaments.

Les échantillons humains sur lesquels cette méthode peut être utilisée sont notamment les érythrocytes, le plasma sanguin, les plaquettes, les globules blancs, le liquide synovial, le liquide céphalo-rachidien, l'urine ou tout extrait tissulaire.

De plus, cette méthode de dosage peut être mise en oeuvre pour effectuer les études de pharmacocinétique nécessaires au développement et à l'utilisation de nouveaux médicaments présentant une activité SOD.

Cette méthode de dosage peut également être utilisée pour le contrôle de qualité de préparations cosmétiques ou pharmaceutiques présentant une activité SOD.

D'une façon générale, cette méthode de dosage peut être utilisée par les chercheurs, sur tout échantillon biologique ou toute solution artificielle susceptible de présenter une activité SOD.

Selon un mode préféré de réalisation, le procédé de dosage de l'activité superoxyde dismutase d'un échantillon, notamment biologique, repose sur une mesure spectrophotométrique de la vitesse d'autoxydation d'un composé de formule générale I ci-dessus, en l'absence et en présence dudit échantillon.

Plus précisément, selon un mode préféré de réalisation, l'invention a pour objet un procédé de dosage de l'activité superoxyde dismutase (SOD) dans un échantillon liquide, caractérisé en ce qu'il comprend essentiellement les étapes

consistant à :

1°) déterminer la vitesse maximale d'autoxydation Vs d'un composé de formule générale I en présence de l'échantillon, au moyen de l'évolution de l'absorbance en fonction du temps, à la longueur d'onde caractérisant l'apparition du produit d'autoxydation du composé de formule générale I utilisé, dans un milieu réactionnel tamponné à une valeur de pH de 8,0 à 9,0, en déclenchant la réaction par ajout d'un aliquot d'une solution-mère du composé de formule générale I ;
2°) déterminer, dans les mêmes conditions, la vitesse maximale d'autoxydation $V_c$ du même composé en l'absence de l'échantillon ; et
3°) déterminer l'activité SOD de l'échantillon au moyen des vitesses $V_s$ et $V_c$ obtenues et d'une courbe d'étalonnage établie dans les mêmes conditions opératoires.

Le milieu réactionnel est avantageusement constitué par un tampon aminé, comme par exemple le 2-amino-2-méthyl-1,3-propanediol (AMPD), contenant de l'acide diéthylènetriaminepenta-acétique (DTPA) 0,1 mM et permettant de fixer le pH à une valeur comprise dans la gamme de 8,0 à 9,0, à la température de mesure, par exemple par un ajout de soude ou d'acide chlorhydrique, selon la nature du tampon considéré.

Ce milieu réactionnel doit être équilibré à la température de travail et saturé d'air à cette température.

La température de travail choisie, par exemple 37°C, doit être maintenue constante pendant les mesures.

La réaction est déclenchée par l'ajout dans le milieu réactionnel (avec ou sans SOD) d'un aliquot d'une solution-mère de l'un des composés de formule générale I dans une solution aqueuse ou dans un solvant organique miscible à l'eau, tel que par exemple l'acétonitrile, le diméthylformamide ou le diméthylsulfoxyde, ou encore dans un mélange d'un tel solvant organique avec de l'eau distillée.

Pour s'affranchir d'une préparation extemporanée de cette solution-mère ou d'une préparation anaérobie de celle-ci, la solution-mère peut être préparée en présence d'un dérivé du bore, tel que l'acide borique par exemple, qui permet d'obtenir une solution de réactif stable pendant une durée qui dépend des concentrations respectives du réactif et du dérivé du bore, de la nature respective du réactif et de ce dérivé et des conditions de stockage de la solution-mère de réactif. Ainsi, une solution du réactif BXT01050 décrit ci-après (exemple 7), préparée dans le mélange diméthylsulfoxyde/eau distillée à 50:50 (volume/volume) contenant de l'acide borique, telle que le rapport des concentrations en acide borique et en réactif soit égal à 150, est stable au moins un mois si la solution est conservée entre 0 et 8°C.

Après homogénéisation de la solution, l'évolution de l'absorbance est enregistrée en fonction du temps, à la longueur d'onde caractérisant l'apparition du produit d'autoxydation du réactif utilisé (par exemple 525 nm pour le réactif BXT01050 et 539 nm pour la braziline), afin de déterminer la vitesse maximale d'autoxydation.

La détermination de l'activité SOD dans un échantillon biologique peut être effectuée en déterminant le rapport ou la différence des vitesses mesurées en présence ($V_s$) et en l'absence de l'échantillon ($V_c$).

Ce rapport ou cette différence peut être reporté par exemple en ordonnée d'une courbe d'étalonnage qui a été établie dans les mêmes conditions opératoires à partir, par exemple, d'un standard de SOD.

Cette courbe d'étalonnage peut être tracée en représentant, en fonction de la concentration du standard utilisé, la variation du rapport ou de la différence des vitesses d'autoxydation mesurées en présence ($V_s$) et en absence de ce standard ($V_c$).

Le rapport $V_s/V_c$ varie avec la concentration en SOD, selon une fonction hyperbolique du type $y = 1+x/(ax+b)$, dans laquelle x représente la concentration en SOD, et a et b sont des constantes. L'inverse de la différence des vitesses en fonction de l'inverse de la concentration en SOD en est une des linéarisations possibles.

Selon l'intensité de l'activité SOD à mesurer, la sensibilité du dosage dans la gamme de pH utilisée (8,0 à 9,0) est adaptable en modifiant par exemple un ou plusieurs des paramètres suivants : concentration en composé de formule générale I choisi, concentration du tampon et température de mesure.

La vitesse d'autoxydation du réactif utilisé peut être modulée également en faisant varier la concentration d'un dérivé du bore dans le milieu réactionnel.

L'existence, dans un échantillon, notamment biologique, de composés interférant avec le dosage se manifeste le plus souvent par une diminution de la vitesse mesurée en présence de l'échantillon, par rapport à celle mesurée en son absence.

Dans le cas d'interférences liées à la présence de mercaptans, tels que par exemple le glutathion, l'introduction dans le milieu réactionnel d'un "réactif piégeur de mercaptans" tel que défini plus haut est alors nécessaire.

Ainsi le tétrafluoroborate de 1,4,6-triméthyl-2-vinylpyridinium (composé de l'exemple 12), par exemple, sera incorporé à une concentration cinquante fois supérieure à celle du glutathion pour le dosage de l'activité SOD dans un lysat érythrocytaire.

Si l'existence, dans un échantillon, notamment biologique, d'un composé susceptible d'introduire une activation artéfactuelle de l'autoxydation du composé de formule générale I, ou composé chromogène, est suspectée, une combinaison telle que celle décrite ci-dessous, qui fait intervenir une seconde mesure effectuée dans des conditions d'inhibition de l'autoxydation, peut être entreprise.

De même, dans le cas de l'exploration de milieux, notamment biologiques, présentant une faible activité SOD, la sensibilité et la précision du dosage peuvent être accrues en combinant à la mesure faite dans des conditions d'activation de l'autoxydation, une mesure effectuée dans des conditions d'inhibition de l'autoxydation.

L'invention a donc également pour objet un procédé du type décrit ci-dessus, caractérisé en ce qu'il comporte également une mesure effectuée dans des conditions d'inhibition de l'autoxydation.

Le milieu réactionnel utilisé pour cette seconde mesure se distingue du milieu décrit précédemment par l'emploi d'un tampon à un pH compris dans la gamme de 7,2 à 7,8, notamment un tampon phosphate constitué par exemple par un mélange adéquat de dihydrogénophosphate de potassium et d'hydrogénophosphate dipotassique, toutes choses étant égales par ailleurs.

On mesure, dans ces conditions opératoires, la vitesse maximale d'autoxydation du composé de formule générale I utilisé en absence ($V'_c$) et en présence de l'échantillon ($V'_s$). La détermination de l'activité SOD dans l'échantillon est alors obtenue au moyen de ($V_s$ - $V_c$ - $V'_s$ + $V'_c$).

Comme précédemment, la valeur obtenue peut être reportée en ordonnée d'une courbe d'étalonnage, qui a été établie dans les mêmes conditions expérimentales et qui donne la variation de ($V_s$-$V_c$- $V'_s$ + $V'_c$) en fonction de la concentration du standard utilisé.

La nouvelle méthode de dosage selon l'invention utilisant comme réactif un composé de formule générale I est, de par son principe, une méthode de mesure spécifique de l'activité SOD. Elle est sensible, rapide et d'une mise en oeuvre aisée, et est de plus entièrement automatisable ; elle est donc adaptée au traitement de grandes séries d'échantillons, comme cela peu être le cas en Chimie Clinique.

De plus, les interférences liées à la présence de mercaptans, fréquemment rencontrés dans les échantillons biologiques, sont facilement éliminées par l'utilisation d'au moins un "réactif piégeur de mercaptans" tel que défini plus haut, sans autre phase supplémentaire de traitement de l'échantillon.

Enfin, les conditions opératoires très modulables de ce procédé de dosage lui confèrent une souplesse d'emploi permettant de l'adapter à des échantillons, notamment biologiques, très divers et donc de prendre en compte les besoins particuliers éventuels de certains utilisateurs, comme cela est le cas en Recherche.

Des exemples d'application du nouveau procédé de dosage de l'activité SOD à l'aide de sept composés de formule générale I, dans le cas d'un lysat érythrocytaire, sont donnés, à titre illustratif, dans la partie expérimentale qui suit.

L'invention a également pour objet un nécessaire ou kit pour la mise en oeuvre du procédé de dosage de l'activité SOD selon l'invention.

Ce nécessaire ou kit comprend essentiellement, en tant que réactif, un composé de formule générale I, telle que définie ci-dessus.

Selon un mode avantageux de réalisation, le nécessaire ou kit pour la mise en oeuvre du procédé de dosage de l'activité SOD selon l'invention comprend en outre un ou plusieurs composé(s) piégeur(s) de mercaptans, de formule générale II, telle que définie ci-cessus.

Selon un mode préféré de réalisation, l'invention a également pour objet un nécessaire ou kit pour la mise en oeuvre du procédé de dosage de l'activité SOD selon l'invention, caractérisé en ce qu'il comprend essentiellement :

- un composé de formule générale I en solution acide ou en poudre,
- un ou plusieurs composé(s) piégeur(s) de mercaptans de formule générale II, en solution ou en poudre, et
- un tampon à base de AMPD, tamponnant dans la gamme de pH de 8,0 à 9,0.

L'invention a en outre pour objet les composés de formule générale I telle que définie précédemment, étant entendu que lorsque n = 1 et $R^2$ = $-CH_2-O-$, $R^1$ et $R^3$ ne peuvent représenter simultanément $-OR^4$, avec $R^4$ = hydrogène.

Les figures annexées représentent les courbes étalonnage pour la mise en oeuvre du procédé selon l'invention en utilisant six composés différents de formule générale I, selon le mode opératoire décrit respectivement dans les exemples 14 à 19 et 21 de la partie expérimentale qui suit.

Ces courbes d'étalonnage ont été obtenues selon deux méthodes différentes, données ici à titre d'exemples, à savoir :

Première méthode :

- en portant l'inverse de la "concentration en SOD", exprimé en ml/U (U = unité d'activité SOD) en abscisse et
- en portant l'inverse de $V_s$-$V_c$, $V_s$ et $V_c$ étant définis comme précédemment, exprimé en min/ΔAbs. (ΔAbs. = variation d'absorbance), en ordonnée.

Deuxième méthode :

- en portant la " concentration en SOD", exprimée en U/ml (U = unité d'activité SOD) en abscisse et
- en portant le rapport $V_s/V_c$, $V_s$ et $V_c$ étant définis comme précédemment, en ordonnée.

Plus précisément :

- la figure 1 représente la courbe d'étalonnage du composé BXTO1041, obtenue selon la première méthode ;
- la figure 2 représente la courbe d'étalonnage du composé BXT01048, obtenue selon la premère méthode ;
- la figure 3 représente la courbe d'étalonnage du composé BXT01049, obtenue selon la première méthode ;
- la figure 4 représente la courbe d'étalonnage du composé BXT01050, obtenue selon la première méthode ;
- la figure 5 représente la courbe d'étalonnage de la braziline, obtenue selon la première méthode ;
- la figure 6 représente la courbe d'étalonnage du brazilane, obtenue selon la première méthode ; et
- la figure 7 représente la courbe d'étalonnage du composé BXT01050, obtenue selon la deuxième méthode.

PARTIE EXPERIMENTALE

I. Préparation de composés de formule générale I :

Toutes les réactions sont effectuées sous atmosphère inerte d'azote, sauf indication contraire.

Les spectres de masse (SM) sont enregistrés sur un appareil R10-10B de la Société Nermag. Le mode d'ionisation utilisé est soit l'impact électronique (IE) à 70 électron-volts, soit l'ionisation chimique (IC), sauf indication contraire.

Les spectres RMN $^1$H sont enregistrés sur un appareil de type Gemini-200 de la Société VARIAN. Les déplacements chimiques sont exprimés en ppm par rapport au tétraméthylsilane. Les multiplicités sont exprimées comme suit : "s" pour singulet; "d" pour doublet; "t" pour triplet ; et "m" pour multiplet.

Les points de fusion (pF) sont enregistrés avec un appareil de la Société Gallenkamp et sont donnés non corrigés. Les solvants de recristallisation sont indiqués entre parenthèses.

A. Composés de la série I.

Exemple 1 : **Préparation du 5,6-dihydroxy-1-(4-hydroxyphényl)indane (BXT01041) :**

5,6-Diméthoxy-3-(4-méthoxyphényl)indène

A une suspension de magnésium (0,24 g, 10 mmol) dans 5 ml de THF anhydre, on ajoute une petite portion de 4-bromoanisole. le mélange est chauffé légèrement pour initier la réaction, puis le reste de dérivé bromé (1,87 g, 10 mmol), en solution dans 5 ml de THF, est ajouté goutte à goutte en 15 min. Après 1 h de reflux, le magnésium a disparu. Le mélange, ainsi obtenu, est refroidi à température ambiante et on ajoute une solution de 5,6-diméthoxyindan-1-one (1,92 g, 10 mmol) dans 10 ml de THF anhydre. La solution brune obtenue est agitée pendant 20 h dans ces conditions puis hydrolysée par addition d'une solution saturée de $NH_4Cl$. On extrait le mélange avec de l'AcOEt. Les phases organiques rassemblées sont lavées avec une solution aqueuse saturée de NaCl et séchées sur $Na_2SO_4$. Après évaporation du solvant, on obtient une huile jaune (3,43 g) qui est reprise par 5 ml d'acide acétique glacial. La solution est agitée 15 min à température ambiante puis chauffée à 70°C pendant 15 min. La solution refroidie à 0°C est versée lentement dans 50 ml d'une solution saturée de $NaHCO_3$. On extrait le mélange avec de l'AcOEt. Les phases organiques rassemblées sont lavées avec une solution aqueuse saturée de $NaHCO_3$, séchées sur $MgSO_4$ et évaporées. Le produit brut (2,84 g) ainsi obtenu est purifié par chromatographie, en éluant avec un mélange AcOEt/hexane (1;3), pour fournir un solide blanc (1,97 g, 70 %).
Caractéristiques physiques :
*pF : 111,0-112,0°C (AcOEt/hexane=1:3).
*RMN $^1$H ($CDCl_3$) : 3,62 (d, J=2,16 Hz, 2H); 4,05 (s, 3H); 4,08 (s, 3H); 4,12 (s, 3H); 6,58 (t, J=2,16 Hz, 1H); 7,19 (d, J=8,67 Hz, 2H); 7,29 (s, 1H); 7,32 (s, 1H); 7,72 (d, J=8, 67 Hz, 2H).
*SM (IE) : 282 (100), 267 (55), 251 (19), 181 (24), 165 (35), 152 (51), 84 (53).

5,6-Diméthoxy-1-(4-méthoxyphényl)indane

Une solution de 5,6-diméthoxy-3-(4-méthoxyphényl)-indène (1,5 g, 5,32 mmol) dans 60 ml d'éthanol est hydrogénée dans un autoclave en présence de palladium sur charbon (à 10 %, 25 mg) sous 5 bars d'hydrogène pendant 1,5 h. Après filtration du catalyseur, l'évaporation du solvant fournit une huile qui se solidifie progressivement (1,5 g, 100 %).

Caractéristiques physiques :
*RMN $^1H$ ($CDCl_3$) : 1,99 (m, 1H); 2,45 (m, 1H); 2, 90 (m, 2H); 3,72 (s, 3H); 3,79 (s, 3H); 3,87 (s, 3H) 4,24 (t, J=7,97 Hz, 1H); 6,48 (s, 1H); 6,82 (s, 1H); 6,84 (d, J=8,70 Hz, 2H); 7,50 (d, J=8,70 Hz, 2H).
*SM (IE) : 284 (100), 269 (26), 253 (59), 241 (17), 177 (21), 165 (35), 152 (28), 115 (28), 84 (25), 77 (26).

5,6-Dihydroxy-1-(4-hydroxyphényl) indane

A une solution du phénylindane précédemment obtenu (500 mg, 0,86 mmol) dans 2 ml de $CH_2Cl_2$, refroidie à -70°C, est ajoutée goutte à goutte une solution de $BBr_3$ dans $CH_2Cl_2$ (1,0 M, 3,1 ml, 3,1 mmol). La solution rouge obtenue est réchauffée à température ambiante et agitée pendant 0,5 h à cette température. Le mélange réactionnel est versé dans un mélange d'eau glacée et d'AcOEt. La phase aqueuse est saturée par NaCl puis extraite deux fois à l'AcOEt. Les phases organiques rassemblées sont lavées avec une solution aqueuse saturée de NaCl, séchées sur $MgSO_4$ et évaporées à sec pour donner une huile jaune (480 mg) qui est chromatographiée (éluant : AcOEt, hexane=1: 1), pour conduire au composé attendu (solide blanc, 400 mg, 93 %).
Caractéristiques physiques :
*RMN $^1H$ (acétone-d6): 1,90 (m, 1H); 2,41 (m, 1H); 2,79 (m, 2H); 4,08 (t, J=7,97 Hz, 1H); 6,39 (s, 1H); 6,75 (s, 1H); 6,77 (d, J=8,70 Hz, 2H); 6,98 (d, J=8,70 Hz, 2H); 7,77 (s, large, 3H).
*SM (IE): 242 (100), 225 (61).

Exemple 2 : **Préparation du 5,6-dihydroxy-1-(4-hydroxyphényl)-2,2-diméthylindane (BXT01045) :**

5,6-Diméthoxy-2-méthylindan-1-one et 5,6-diméthoxy-2,2-diméthylindan-1-one

Un mélange de diisopropylamidure de lithium (LDA, 1,5 M dans du cyclohexane, 7,5 ml, 11,3 mmol) et de 15 ml de THF anhydre est refroidi à -78°C. On y ajoute goutte à goutte une solution de 5,6-diméthoxyindan-1-one (1,92 g, 10 mmol) dans 5 ml de THF. Le mélange est agité 30 min à -78°C. On ajoute de l'iodure de méthyle (0,93 ml, 15 mmol). Après 30 min à -78°C, la solution est réchauffée à température ambiante et agitée à cette température pendant 4 h. On refroidit à nouveau la solution à -78°C et ajoute goutte à goutte la même quantité de LDA que précédemment. Après 30 min, on ajoute du MeI (0,93 ml 15 mmol). On agite 30 min à -78°C puis 4 h à température ambiante. Le mélange réactionnel est hydrolysé par addition d'une solution aqueuse saturée de $NH_4Cl$. On extrait avec de l'AcOEt. Les phases organiques sont lavées avec une solution aqueuse saturée de NaCl, séchées sur $MgSO_4$ et évaporées à sec pour donner un solide jaune (2,23 g). Une chromatographie, en utilisant un mélange AcOET/hexane (1:9) comme éluant, donne la 5,6-diméthoxy-2,2-diméthylindan-1-one (1,35 g, 61 %) et la 5,6-diméthoxy-2-méthylindan-1-one (0,43 g, 21, %).
Caractéristiques physiques de la 5,6-diméthoxy-2,2-diméthylindan-1-one :
*RMN $^1H$ ($CDCl_3$) : 1,64 (s, 6H); 2,85 (s, 2H); 3,85 (s, 3H); 3,91 (s, 3H); 6,80 (s, 1H); 7,12 (s, 1H).
*pF : 134,0-134,5°C (EtOH).
Caractéristiques physiques de la 5,6-diméthoxy-2-méthylindan-1-one :
*RMN $^1H$ ($CDCl_3$); 1,22 (d, J=7,41 Hz, 3H); 2,63 (m,2H); 3,23 (dd, J=9,24-16,85 Hz, 1H); 3,84 (s, 3H); 3,90 (s, 3H); 6,81 (s, 1H); 7,11 (s, 1H).
*pF : 106,0-106,5°C (EtOH).

1-Hydroxy-5,6-diméthoxy-1-(4-méthoxyphényl)-2,2-diméthylindane

A une suspension de magnésium (72 mg, 3 mmol) dans 6 ml de THF anhydre, est ajoutée une petite portion de 4-bromoanisole. Le mélange est chauffé au léger reflux pour initier la réaction, puis le reste de dérivé bromé (0,56 g, 3 mmol), en solution dans 5 ml de THF, est ajouté goutte à goutte en 15 min. Après 1 h de reflux, le magnésium est consommé. De la 5,6-diméthoxy-2,2-diméthylindan-1-one (0,66 g, 3 mmol) est ajoutée par petites portions. La solution est agitée 1 h à température ambiante, puis chauffée au reflux 4 h. 10 ml d'une solution aqueuse saturée de $NH_4Cl$ sont ajoutés à 0°C. On extrait le mélange avec de l'AcOEt. les phases organiques rassemblées sont lavées avec une solution aqueuse saturée ce NaCl, séchées sur $MgSO_4$ et évaporées pour donner une huile jaune (1,20 g). Une purification par chromatographie (éluant:AcOEt/hexane=1:5) permet d'isoler le produit attendu (0,65 g, 66 %).
Caractéristiques physiques :
*RMN $^1H$ ($CDCl_3$) : 0,62 (s, 3H); 1,13 (s, 3H); 2,60 (d, J=15,10 Hz, 1H); 2,86 (d, J=15,10 Hz, 1H); 3,77 (s, 3H); 3,79 (s, 3H); 3,89 (s, 3H); 3,91 (s, 1H); 6,71 (s, 1H); 6,79 (s, 1H); 6,83 (d, J=9,00 Hz, 2H); 7,19 (d, J=9,00 Hz, 2H).

5,6-Diméthoxy-1-(4-méthoxythényl)-2,2-diméthylindane

Du borohydrure de sodium (1,40 g, 36,7 mmol) et du trichlorure d'aluminium (0,82 g, 6,1 mmol) sont ajoutés à une solution du précédent produit (0,39 g, 1,25 mmol) dans 15 ml de THF anhydre. Le mélange est chauffé au reflux. Il se colore immédiatement en rose, puis en rouge et finalement devient incolore. Après 20 h de reflux, le mélange réactionnel est versé dans un mélange de 20 g de glace et 5 ml de HCl (1N). On sature la phase aqueuse avec NaCl et on l'extrait avec AcOEt. Les phases organiques sont lavées avec une solution aqueuse saturée de NaCl, séchées sur $MgSO_4$ et évaporées à sec pour fournir une huile incolore. Une chromatographie, en éluant avec un mélange AcOEt-hexane (1;10), donne le produit attendu (0,39 g, 66 %).
Caractéristiques physiques
*RMN $^1H$ ($CDCl_3$) : 0,63 (s, 3H); 1,19 (s, 3H); 2,72 (s, 2H); 3,74 (s, 3H); 3,79 (s, 3H); 3,87 (s, 3H); 3, 91 (s, 1H); 6,56 (s, 1H); 6,78 (s, 1H); 6,83 (d, J=8,86 Hz, 2H); 6,98 (d, J=8,86 Hz, 2H).

5,6-Dihydroxy-1-(4-hydroxyphényl)-2,2-diméthylindane

A une solution du dérivé précédemment obtenu (0,39 g, 1,25 mmol) dans 2 ml de $CH_2Cl_2$, refroidie à -70°C, on ajoute goutte à goutte une solution de $BBr_3$ dans $CH_2Cl_2$ (1,0 M, 4, 86 ml, 4, 86 mmol). La solution rouge obtenue est réchauffée à température ambiante et on l'agite pendant 1 h à cette température. Le mélange réactionnel est verse dans un mélange d'eau glacée et d'AcOEt. La phase aqueuse est saturée par NaCl, puis extraite deux fois à l'AcOEt. les phases organiques rassemblées sont lavées avec une solution aqueuse saturée de NaCl, séchées sur $MgSO_4$ et évaporées à sec, pour donner une huile légèrement rouge (410 mg) qui est chromatographiée (éluant : AcOEt/hexane=1:1), pour conduire au composé attendu (0,29 g, 86 %).
Caractéristiques physiques :
*RMN $^1H$ (acétone-d6) : 0,71 (s, 3H); 1,17 (s, 3H); 2,60 (s, 2H); 3,80 (s, 1H); 6,46 (s, 1H); 6,72 (s, 1H); 6,75 (d, J=8,74 Hz, 2H); 6,89 (d, J=8,74 Hz, 2H).
*SM (IE) : 270 (87), 227 (100), 107 (16).

Exemple 3 : **Préparation du 5,6-dihydroxy-1-(4-hydroxyphényl)-2-méthylindane (BXT01048) :**

5,6-Diméthoxy-3-(4,-méthoxythényl)-2-méthylindène

A une suspension de magnésium (42 mg, 1,750 mmol) dans 5 ml de THF anhydre, est ajoutée une petite portion de 4-bromoanisole. Le mélange est chauffé au léger reflux pour initier la réaction puis le reste de dérivé bromé (330 mg, 1,75 mmol), en solution dans 5 ml de THF, est ajouté goutte à goutte en 15 min. Après 1 h de reflux, le magnésium est consommé. Le mélange, ainsi obtenu, est refroidi à température ambiante puis on ajoute une solution de 5,6-diméthoxy-2-méthylindan-1-one (300 mg, 1,46 mmol) dans 5 ml de THF. La solution brune obtenue est agitée pendant 18 h, puis hydrolysée par addition d'une solution aqueuse saturée de $NH_4Cl$. On extrait le mélange avec de l'AcOEt. Les phases organiques rassemblées sont lavées avec une solution aqueuse saturée de NaCl, puis séchées sur $Na_2SO_4$. Après évaporation du solvant, on obtient une huile jaune (0,54 g) qui est reprise par 2 ml d'acide acétique glacial. La solution obtenue est agitée 1 h à température ambiante. La solution, refroidie à 0°C, est versée lentement dans 50 ml d'une solution saturée de $NaHCO_3$. On extrait le mélange avec de l'AcOEt. La phase organique est lavée avec une solution aqueuse saturée de $NaHCO_3$, séchée sur $MgSO_4$ et évaporée. Le produit brut (530 mg) ainsi obtenu est purifié par chromatographie, en éluant avec un mélange AcOEt/hexane (1:9), pour fournir une huile incolore (0,25 g, 58 %).
Caractéristiques physiques :
*RMN $^1H$ ($CDCl_3$) : 2,09 (s, 3H); 3,35 (s, 2H); 3,82 (s, 3H); 3,86 (s, 3H); 3,89 (s, 3H); 6,78 (s, 1H); 7,00 (d, J=8,70 Hz, 2H); 7,04 (s, 1H); 7,33 (d, J=8,70 Hz, 2H).

5,6-Diméthoxy-1-(4-méthoxyphényl)-2-méthylindane

Une solution du phénylindène précédent (240 mg, 0,81 mmol) dans 5 ml d'AcOEt est hydrogénée sous $10^6$ Pa environ (10 atm) d'hydrogène, en présence de 20 mg de palladium sur charbon (à 10 %) pendant 2 h. Le catalyseur est éliminé par filtration, puis le filtrat est évaporé à sec, pour donner le produit attendu pur (0,24 g, 99 %).
Caractéristiques physiques :
*RMN $^1H$ ($CDCl_3$) : 0,69 (d, J=6,96 Hz, 3H); 2,58 (dd, J=6,96-14,30 Hz, 1H); 2,80 (m, 1H); 2,96 (dd, J=7,04-14,30 Hz, 1H); 3,75 (s, 3H); 3,77 (s, 3H); 3,88 (s, 3H); 4,25 (d, J=7,68 Hz, 1H): 6,63 (s, 1H); 6,80 (d, J=8,78 Hz, 2H); 6,81 (s, 1H); 6,88 (d, J=8,78 Hz, 2H).
*SM (IE) : 298 (100), 283 (25), 267 (44), 252 (7); 238 (12), 167 (42), 149 (42), 135 (33); 84 (62).

5,6-Dihydroxy-1-(4-hydroxyphényl)-2-méthylindane

A une solution du phénylindane précédemment obtenu (0,23 g, 0,77 mmol) dans 2 ml de $CH_2Cl_2$, refroidie à -70°C, est ajoutée goutte à goutte une solution de $BBr_3$ dans $CH_2Cl_2$ (1,0 M, 3,0 ml, 3,0 mmol). La solution rouge obtenue est réchauffée à température ambiante et agitée pendant 0,5 h à cette température. Le mélange réactionnel est versé dans un mélange d'eau glacée et d'AcOEt. La phase aqueuse est saturée par NaCl, puis extraite deux fois à l'AcOEt. Les phases organiques rassemblées sont lavées avec une solution aqueuse saturée de NaCl, séchées sur $MgSO_4$ et évaporées à sec, pour donner une huile jaune (0,20 g) qui est chromatographiée (éluant : AcOEt/hexane=1:1), pour conduire au composé attendu (0,18 g, 91 %).
Caractéristiques physiques :
*RMN $^1$H (acétone-d6) : 0,63 (d, J=6,96 Hz, 3H); 2,45 (dd, J=7,32-14,22 Hz, 1H); 2,68 (m, 1H); 2,84 (dd, J=7,04-14,22 Hz, 1H); 4,13 (d, J=7,42 Hz, 1H); 6,50 (s, 1H); 6,73 (m, 5H); 7,53 (s, 1H); 7,55 (s, 1H); 8,06 (s, 1H).
*SM (IE) : 256 (100), 241 (23), 239 (46), 227 (27).

Exemple 4 : **Préparation du 1-(4-diméthylaminophényl)-5,6-dihydroxyindane (BXT01049)** :

5,6-Dihydroxyindan-1-one

De la 5,6-diméthylindan-1-one (3,13 g; 16,3 mmol) est dissoute dans 10 ml de dichlorométhane séché sur tamis 3A. A cette solution, jaune clair, est ajoutée, à -70°C, une solution de tribromure de bore dans le dichlorométhane (1M; 28 ml, 28 mmol), goutte à goutte. Le milieu réactionnel vire au bordeaux. La température du milieu est ramenée à température ambiante, puis la solution est agitée pendant 2 h 30. Le milieu réactionnel est versé dans 50 g de glace, puis extrait avec de l'acétate d'éthyle. Les phases organiques sont lavées avec une solution aqueuse saturée en NaCl, puis séchées sur $MgSO_4$. Le solvant est évaporé sous pression réduite.

Le produit brut (2,3 g; 87 %) est utilisé tel quel pour l'étape suivante.

5,6-Di(terbutyldiméthylsilyloxy)indan-1-one

A une solution de la 5,6-dihydroxyindan-1-one précédemment décrite (2 g; 12,2 mmol), dans 40 ml de DMF séché sur tamis 3A, refroidie à 0°C, on ajoute du chlorure de terbutyldiméthylsilyle (7,35 g; 49,8 mmol) et de l'imidazole (3,82 g; 56,1 mmol). La température est maintenue 10 min à 0°C, puis 16 h à température ambiante. Le produit est extrait avec de l'acétate d'éthyle. La phase organique est lavée avec une solution aqueuse saturée en NaCl, puis séchée sur $MgSO_4$. Le solvant est évaporé sous pression réduite. Le produit désiré est purifié par chromatographie avec un éluant : hexane/acétate d'éthyle (8:2) et obtenu sous forme d'un solide beige (4,5 g; 94 %). Caractéristiques physiques :
*RMN $^1$H ($CDCl_3$) : 0,195 (s, 6H); 0,205 (s, 6H); 0,92 (s, 18H); 2,60 (t, J=7,63 Hz, 2H); 2,95 (t, J=7,63 Hz, 2H); 6,85 (s, 1H); 7,15 (s, 1H).

5,6-Di(terbutyldiméthylsilyloxy)-3-(4-diméthylaminophényl)indène

Du magnésium (124 mg, 5,1 mmol) est recouvert par 1 ml de THF distillé et chauffé à 60°C sous agitation. Une solution de 4-bromo-N,N-diméthylaniline (1,02 g, 5,1 mmol) dans 5 ml de THF distillé est ajoutée goutte à goutte en 20 min. Le mélange réactionnel est chauffé au reflux du THF pendant 1 h. A cette solution jaune vert, on ajoute une solution de 5,6-di(terbutyldiméthylsilyloxy)indan-1-one (2 g, 5,1 mmol) dans 10 ml de THF distillé, goutte à goutte, en 5 min, à température ambiante. Le milieu est porté au reflux pendant 20 h. le mélange réactionnel, de coloration violette, est hydrolysé par une solution aqueuse saturée en chlorure d'ammonium, puis extrait avec de l'acétate d'éthyle. La phase organique est lavée avec une solution aqueuse saturée en NaCl, puis séchée sur $MgSO_4$. le solvant est évaporé sous pression réduite. Le produit désiré est purifié par chromatographie avec un éluant : hexane/acétate d'éthyle (9:1) et obtenu sous forme d'une huile (0,9 g; 36 %).
Caractéristiques physiques :
*RMN $^1$H ($CDCl_3$) : 0,16 (s, 12H); 0,95 (s, 18H); 2,94 (s, 6H); 3,50 (d, J=2,03 Hz, 2H); 6,30 (t, J=2,03 Hz, 1H); 6,75 (m, 2H); 6,95 (s, 1H); 7,08 (s, 1H); 7,45 (d, J=7,85 Hz, 2H).

5,6-Di(terbutyldiméthylsilyloxy)-1-(4-diméthylaminophényl)indane

Une solution du composé précédent (0,9 g; 1,82 mmol), dans 10 ml de méthanol et 2 ml d'acétone, est agitée en présence de palladium sur charbon (à 10 %, 60 mg), à température ambiante sous pression d'hydrogène d'environ $5.10^5$ Pa (5 bars), pendant 3 h. La suspension est filtrée, puis le filtrat est évaporé à sec sous pression réduite. Le produit désiré est purifié par chromatographie avec un éluant : hexane/acétate d'éthyle (9:1) et obtenu sous forme

d'un solide beige clair (0,64 g; 71 %).
Caractéristiques physiques :
*RMN $^1$H ($CDCl_3$) : 0,08 (s, 6H); 0,18 (s, 6H); 0,90 (s, 9H); 0,95 (s, 9H); 1,90 (m, 2H); 2,45 (m, 2H); 2,90 (s, 6H); 4,17 (t, J=8,07 Hz, 1H); 6,4 (s, 1H); 6,7 (m, 2H); 7,05 (m, 2H); 7,20 (s, 1H).

5,6-Dihydroxy-1-(4-diméthylaminophényl)indane

Le produit précédemment obtenu (0,56 g; 1,12 mmol) est dissous dans 5 ml de THF distillé, puis additionné d'une solution molaire de fluorure de tétrabutylammonium (2,26 ml; 2,26 mmol) dans le THF, sous agitation et à température ambiante. L'agitation est maintenue pendant 1 h à cette température. Après addition d'eau, le produit est extrait avec de l'acétate d'éthyle. La phase organique est lavée avec une solution aqueuse saturée en NaCl, puis séchée sur $MgSO_4$. Le solvant est évaporé sous pression réduite. Le produit désiré est purifié par une double chromatographie :
1ère purification: éluant: hexane-acétate d'éthyle (6:4)
2ème purification: éluant: dichlorométhane-méthanol (95:5).

Le produit désiré est obtenu sous forme d'une huile mauve (190 mg; 62 %).
Caractéristiques physiques :
*RMN $^1$H ($CDCl_3$) : 1,96 (m, 2H); 2,44 (m, 2H); 2,89 (s, 6H); 4,1 (t, J=7,2 Hz, 1H); 6,4 (s, 1H); 6,73 (d, J=6,7 Hz, 2H); 6,77 (s, 1H); 7,05 (d, J=6,7 Hz, 2H).
*SM (IE) : 269 (100), 254 (10), 240 (9), 225 (28), 77 (10).
*CLHP (chromatographie liquide haute performance) : (colonne : phase inverse RP18; longueur : 15 cm; éluant : dichlorométhane-méthanol (94:6); débit : 1 ml/min, longueur d'onde d'observation : 254 nm); temps de rétention : 2,37 min; pureté : 99,1 %.

B. Composés de la série II :

Exemple 5 : **Préparation du 2,5,6-trihydroxy-1-(4-hydroxyphényl)indane (BXT01035) :**

2-Hydroxy-5,6-diméthoxy-1-(4-méthoxyphényl)indane

A une solution de 5,6-diméthoxy-3-(4-méthoxyphényl)indène (1,0 g, 3,55 mmol) dans 10 ml de THF anhydre, on ajoute à 0°C, une solution de $BH_3$.THF (1,0 M, 4,26 ml, 4,26 mmol), en 15 min. Le mélange obtenu est agité 1 h à la même température puis 4 h à température ambiante. La solution est refroidie à 0°C et 3,5 ml d'eau sont ajoutés goutte à goutte, puis 7 ml de NaOH (à 10 %). On ajoute ensuite une solution aqueuse saturée de $H_2O_2$ (à 30 %, 7 ml) et on agite le mélange pendant 2 h. On extrait le mélange avec 50 ml d'AcOEt. La phase organique est lavée avec 10 ml d'eau, puis séchée sur $MgSO_4$. L'évaporation du solvant donne un solide blanc (1,16 g) qui est purifié par chromatographie, en éluant avec un mélange AcOEt/hexane (1:3), pour fournir le produit attendu (solide blanc, 0,97 g, 91 %).
Caractéristiques physiques :
*pF : 128,0-129,0°C (AcOEt/hexane=1:4).
*RMN $^1$H ($CDCl_3$) : 1,96 (d, J=4,68 Hz, 1H, échangeable avec $D_2O$); 2,84 (dd, J=6,79-15,39 Hz, 1H); 3,21 (dd, J=6,92-15,39 Hz, 1H); 3,73 (s, 3H); 3,79 (s, 3H); 3,87 (s, 3H); 4,05 (d, J=6,23 Hz, 1H); 4,39 (m, 1H); 6,46 (s, 1H); 6,81 (s, 1H); 6,87 (d, J=8,64 Hz, 2H); 7,07 (d, J=8,64 Hz, 2H).
*SM (IE) : 300 (100), 282 (18), 257 (27), 239 (5), 165 (7), 121 (15).

2,5,6-Trihydroxy-1-(4-hydroxyphényl)indane

Du tribromure d'aluminium (2,67 g, 10,0 mmol) est mélangé avec 24 ml d'éthanethiol. Le phénylindane précédent (300 mg, 1,0 mmol) est ajouté en une seule portion. Après 62 h à température ambiante, l'éthanethiol est évaporé, puis le résidu est traité avec 15 ml d'eau à 0°C. Le mélange est extrait avec de l'AcOEt. Les phases organiques rassemblées sont lavées avec une solution aqueuse saturée de NaCl, séchées sur $Na_2SO_4$ et évaporées à sec pour conduire à une huile jaune (250 mg). Une purification par chromatographie, en utilisant un mélange AcOEt/hexane (1: 1) comme éluant, donne le produit attendu (solide blanc, 140 mg, 54 %).
Caractéristiques physiques :
*RMN $^1$H (acétone-d6) : 2,68 (dd, J=6,31-15,02 Hz, 1H); 3,06 (dd, J=6,59-15,30 Hz, 1H); 3,90 (d, J=6,53 Hz, 1H); 4,20 (d, J=5,28 Hz, 1H); 4,29 (m, 1H); 6,30 (s, 1H); 6,68 (s, 1H); 6,76 (d, J=8,47 Hz, 2H); 6,96 (d, J=8,47 Hz, 2H); 7,57 (s, 1H); 7,63 (s, 1H); 8,18 (s, 1H).
*SM (IE) : 258 (100), 240 (40), 229 (13), 215 (39), 183 (27), 165 (32), 107 (46), 77 (28).

Exemple 6 : **Préparation du 5,6-dihydroxy-1-(4-hydroxyphényl)-2-méthoxyindane (BXT01040)** :

5,6-Dihydroxy-3-(4-hydroxyphényl)indène

A une solution de 5,6-diméthoxy-3-(4-méthoxyphényl)indène (0,60 g, 2,23 mmol) dans 2 ml de $CH_2Cl_2$, refroidie à -70°C, on ajoute goutte à goutte une solution de $BBr_3$ dans $CH_2Cl_2$ (1,0 M, 9,60 ml, 9,60 mmol). La solution rouge obtenue est réchauffée à température ambiante et agitée pendant 1 h à cette température. Le mélange réactionnel est versé dans un mélange d'eau glacée et d'AcOEt. La phase aqueuse est saturée par NaCl, puis extraite deux fois à l'AcOEt. Les phases organiques rassemblées sont lavées avec une solution aqueuse saturée de NaCl, séchées sur $MgSO_4$ et évaporées à sec, pour donner un solide rouge (0,62 g) qui est utilise directement dans l'étape suivante.
Caractéristiques physiques :
*RMN $^1H$ (acétone-d6) : 3,28 (d, J=2,10 Hz, 2H); 6,31 (t, J=2,10 Hz, 1H); 6,88 (d, J=8,45 Hz, 2H); 7,03 (s, 1H); 7,07 (s, 1H); 7,44 (d, J=8,45 Hz, 2H); 7,72 (s large, 2H); 8,53 (s large, 1H) .
*SM (IE) : 240 (96), 222 (7), 194 (32), 181 (15), 165 (27), 152 (14), 43 (100).

5,6-Dibenzyloxy-1-(4-benzyloxyphényl)indène

Le produit brut de l'étape précédente (0,62 g) est dissous dans 5 ml d'acétone. On y ajoute du $K_2CO_3$ (1,85 g, 13,38 mmol) et du bromure de benzyle (1,73 g, 10,0 mmol). Le mélange est chauffé au reflux pendant 18 h. Le solide est filtré, puis le filtrat est évaporé à sec. Le résidu est directement chromatographié en éluant avec un mélange AcOEt/hexane (1:5), pour donner le produit attendu (0,82 g) qui est utilisé directement dans l'étape suivante.
Caractéristiques physiques :
*RMN $^1H$ ($CDCl_3$) : 3,36 (d, J=1,83 Hz, 2H); 5,11 (s, 2H); 5,13 (s, 2H); 5,17 (s, 2H); 6,37 (t, J=1,83 Hz, 1H); 7,01 (d, J=8,72 Hz, 2H); 7,17 (s, 1H); 7,25-7,60 (m, 18H).

5,6-Dibenzyloxy-1-(4-benzyloxyphényl)-2-hydroxyindane

A une solution du phénylindène ci-dessus (0,82 g, 1,61 mmol) dans 8 ml de THF anhydre, est ajoutée, à température ambiante, une solution de $BH_3$.THF (1,0 M, 3,20 ml, 3,20 mmol), en 15 min. Le mélange obtenu est agité 3,5 h à température ambiante. La solution est refroidie à 0°C et 3,5 ml d'eau sont ajoutés goutte à goutte, puis 7 ml de NaOH (à 10 %). On ajoute ensuite une solution aqueuse de $H_2O_2$ (à 30 %, 7 ml) et on agite le mélange pendant 2 h. On extrait le mélange avec de l'AcOEt. La phase organique est lavée à l'eau, puis séchée sur $MgSO_4$. L'évaporation du solvant donne une huile incolore (0,95 g) qui est purifiée par chromatographie, en éluant avec un mélange AcOEt/hexane (1:4), pour fournir le produit attendu (solide blanc, 0,46 g, 54 %).
Caractéristiques physiques :
*RMN $^1H$ ($CDCl_3$) : 2,02 (s large, échangeable avec $D_2O$, 1H); 2,81 (dd, J=7,00-15,26 Hz, 1H); 3,17 (dd, J=6,64-15,26 Hz, 1H); 4,03 (d, J=6,28 Hz, 1H); 4,34 (m, 1H); 5,01 (s, 2H); 5,06 (s, 2H); 5,15 (s, 2H): 6,57 (s, 1H); 6,86 (s, 1H); 6,92 (d, J=8,72 Hz, 2H); 7,40 (d, J=8,72 Hz, 2H); 7,24-7,50 (m, 15H).
*SM (IC, $NH_3$) : 546 ($MNH_4^+$).

5,6-Dibenzyloxy-1-(4-benzyloxyphényl)-2-méthoxyindane

On mélange l'alcool précédent (0,26 g, 0,49 mmol) avec de la potasse en poudre (0,11 g, 1,97 mmol). On y ajoute 3 ml de DMSO suivi immédiatement d'iodure de méthyle (0,14 g, 9,85 mmol). Le mélange est agité à température ambiante pendant 3 h. 5 ml d'eau sont ajoutés. Le mélange est extrait avec de l'AcOEt. Les phases organiques rassemblées sont lavées avec une solution aqueuse saturée de NaCl, séchées sur $MgSO_4$ et évaporées pour donner une huile jaune. La purification de cette huile par chromatographie (éluant : AcOEt/hexane=1:9) fournit le produit attendu (0,26 g, 97 %).
Caractéristiques physiques
*RMN $^1H$ ($CDCl_3$) : 2,85 (dd, J=7,01-15,35 Hz, 1H); 3,23 (dd, J=6,78-15,35 Hz, 1H); 3,38 (s, 3H); 4,04 (m, 1H); 4,12 (d, J=6,25 Hz, 1H); 5,03 (s, 2H); 5,08 (s, 2H); 5,14 (s, 2H); 6,60 (s, 1H); 6,88 (s, 1H); 6,93 (d, J=8,64 Hz, 2H); 7,10 (d, J=8,64 Hz, 2H); 7,30-7,60 (m, 15H).

5,6-Dihydroxy-1-(4-hydroxyphényl)-2-méthoxyindane

Une solution du composé précédent (0,26 g, 0,48 mmol) dans 10 ml d'AcOEt est hydrogenée sous environ $10^5$ Pa (1 atm) d'hydrogéne en présence de palladium sur charbon (à 10 %, 10 mg). La réaction est terminée après 12 h. On filtre le catalyseur, puis le solvant est évaporé sous pression réduite pour donner une huile jaune. Ce produit brut

est purifié par chromatographie (éluant : AcOEt/hexane = 1:4), pour fournir le produit attendu (solide blanc : 92 mg, 71 %).
Caractéristiques physiques :
*RMN $^1$H (acétone-d6) : 2,68 (dd, J=6,15-15,62 Hz, 1H); 3,15 (dd, J=6,25-15,62 Hz, 1H); 3,24 (s, 3H); 4,01 (m, 1H); 4,04 (d, J=6,25 Hz, 1H); 6,29 (s, 1H); 6,70 (s, 1H); 6,76 (d, J=8,24 Hz, 2H); 6,98 (d, J=8,24 Hz, 2H), 7,62 (s large, 2H); 8,17 (s large, 1H).
*SM (IE) : 272 (100), 240 (81), 227 (18).

C. Composés de la série III :

Exemple 7 : **Préparation du 5,6,6a,11b-tétrahydro-3,9,10-trihydroxybenzo[c]fluorène (BXT01050) :**

2-(3,4-Diméthoxybenzylidène)-6-méthoxy-1-tétralone

On mélange de la 6-méthoxy-1-tétralone (7,04 g, 40 mmol) et du 3,4-diméthoxybenzaldéhyde (6,44 g, 40 mmol) avec 100 ml d'éthanol absolu. On agite pendant 0,5 h pour dissoudre le maximum de réactifs. On commence à introduire du chlorure d'hydrogène gazeux. On constate une réaction exothermique qui permet de dissoudre complètement tous les réactifs. On refroidit de temps en temps le ballon avec un bain de glace pour que la température ne dépasse pas 80°C. Le mélange est ainsi saturé de chlorure d'hydrogène au bout d'1 h pour donner une couleur rouge intense. On laisse reposer ce mélange à température ambiante pendant 2 h. On verse le mélange réactionnel dans un mélange de 400 ml d'eau et 40 ml de NaOH aqueux (3 N). On agite vigoureusement pendant 3 h à température ambiante. Le solide précipité est filtré sur verre fritté, lavé à l'eau et finalement séché sous vide, sur $P_2O_5$, pour donner un solide (12,35 g, 95 %). Une petite quantité de produit est recristallisée dans le mélange AcOEt/hexane=1:2.
Caractéristiques physiques :
*RMN $^1$H ($CDCl_3$) : 2,89 (t, J=6,96 Hz, 2H); 3,12 (dt, J=1,70-6,96 Hz, 2H), 3,85 (s, 3H); 3,89 (s, 3H); 3, 90 (s, 3H); 6,68 (d, J=2,48 Hz, 1H); 6,85 (dd, J=2,48-8,67 Hz, 1H); 6,88 (d, J=8,14 Hz, 1H); 6,95 (d, J=1,64 Hz, 1H); 7,04 (dd, J=1,64-8,14 Hz, 1H); 7,78 (s, 1H); 8,08 (d, J=8,67 Hz, 1H).
*SM (IE) : 324 (87), 323 (100), 309 (38), 293 (39), 281 (11).
*pF : 109,0-110,0°C (AcOEt/hexane=1:2) .

2-(3,4-Diméthoxybenzyl)-6-méthoxy-1-tétralone

A une solution du composé précédent (35,0 g, 0,107 mol) dans 550 ml d'AcOEt, sont ajoutés 2,0 g de palladium sur charbon (à 10 %). Le mélange est hydrogéné sous environ $0.10^5$ Pa (2 atm) d'hydrogéne pendant 1 h. Le catalyseur est éliminé par filtration et le filtrat est évaporé à sec, pour donner 38,4 g d'un produit brut qui est dissous à chaud dans 100 ml d'AcOEt. On ajoute 130 mi d'hexane. On filtre les cristaux formés (23,56 g). Le filtrat est évaporé à sec. Le résidu est chromatographié en éluant avec un mélange AcOEt/hexane (1:4), pour donner 6,03 g du produit attendu, pur.

Rendement :29,59 g (84 %).
Caractéristiques physiques :
*pF : 101,0-102,0°C (AcOEt/hexane=1:2).
*RMN $^1$H ($CDCl_3$) : 1,67-1,83 (m, 1H); 2,00-2,14 (m, 1H); 2,51-2,72 (m, 2H); 2,84-2,96 (m, 2H); 3,34-3,48 (m, 1H); 3,82 (s, 3H); 3,84 (s, 3H); 3,85 (s, 3H) ; 6,64 (d, J=2,36 Hz, 1H); 6,74-6,84 (m, 4H); 8,02 (d, J=8,74 Hz, 1H).
*SM (IE) : 326 (25), 175 (9), 151 (100), 107 (13), 91 (17), 77 (11).

5,6-Dihydro-3,9,10-triméthoxybenzo[c]fluorène

15,0 g d'acide polyphosphorique sont mélangés avec la tétralone obtenue précédemment (1,04 g, 3,2 mmol), puis chauffés à 80-90°C pendant 4 h. Après avoir refroidi le mélange à température ambiante, 10 g d'eau glacée et 20 ml d'AcOEt sont ajoutés. On sépare la phase organique et extrait la phase aqueuse avec de l'AcOEt. Les phases organiques rassemblées sont lavées avec une solution aqueuse saturée de $NaHCO_3$ puis avec NaCl (sat.), séchées sur $MgSO_4$ et évaporées à sec, pour donner un solide (0,91 g) qui est purifié par chromatographie en éluant avec un mélange AcOEt/hexane (1:6). On obtient le produit attendu (0,61 g) et le produit de départ (0,24 g). le rendement de cette réaction, par rapport au produit consommé, est de 81 %.
Caractéristiques physiques :
*RMN $^1$H ($CDCl_3$) : 2,59 (t, J=7,77 Hz, 2H); 2,89 (t, J=7,77 Hz, 2H); 3,39 (s, 2H); 3,84 (s, 3H); 3,90 (s, 3H); 3,94 (s, 3H); 6,84 (m, 2H); 7,08 (s, 1H); 7,38 (s, 1H); 7,72 (d, j=9,24 Hz, 1H).
*pF : 146,5-147,0°C (AcOEt/hexane=1:4).

*SM (IE) : 308 (100), 293 (14), 277 (38), 202 (24), 176 (33), 165 (17), 152 (21).

5,6,6a,11b-Tétrahydro-3,9,10-triméthoxybenzo[c]fluorène

A une solution du précédent produit (0,90 g, 2,92 mmol) dans 30 ml d'AcOEt, on ajoute 0,15 g de palladium sur charbon (à 10 %). Le mélange est hydrogéné sous une pression d'environ $8.10^5$ Pa (8 atm) d'hydrogène pendant 5 h. Le catalyseur est éliminé par filtration et le filtrat est évaporé à sec, pour donner le produit attendu, pur (0,90 g, 99 %).
Caractéristiques physiques :
*pF : 77,0-79,0°C (AcOEt/hexane=1:4).
*RMN $^1$H ($CDCl_3$) : 1,45-1,65 (m, 1H); 1,66-1,80 (m, 1H); 2,75 (dd, J=6,96-15,10 Hz, 1H); 2,68-2,87 (m, 3H); 3,15 (dd, J=6,96-15,10 Hz, 1H); 3,79 (s, 3H); 3,80 (s, 3H); 3,84 (s, 3H); 4,21 (d, J=6,68 Hz, 1H); 6,66 (d, J=2,64 Hz, 1H; 6,76 (s, 1H); 6,78 (s, 1H); 6,83 (dd, J=2,64-8,38 Hz, 1H); 7,33 (d, J=8,38 Hz, 1H).
*SM (IE) : 310 (100), 295 (23), 279 (46), 267 (15), 189 (24), 121 (23).

5,6,6a,11b-Tétrahydro-3,9,10-trihydroxybenzo[c]fluoruène

A une solution du composé précédemment obtenu (0,57 g, 1,84 mmoi) dans 4 ml de $CH_2Cl_2$, refroidie à -70°C, on ajoute goutte à goutte une solution de $BBr_3$ dans $CH_2Cl_2$ (1,0 M, 6,5 ml, 6,5 mmol). La solution rouge obtenue est réchauffée à température ambiante et agitée pendant 0,5 h à cette température. Le mélange réactionnel est versé dans un mélange d'eau glacée et d'AcOEt. La phase aqueuse est saturée par NaCl, puis extraite deux fois à l'AcOEt. Les phases organiques rassemblées sont lavées avec une solution aqueuse saturée de NaCl, séchées sur $MgSO_4$ et évaporées à sec, pour donner une huile jaune (0,50 g). Une chromatographie de 0,28 g de cette huile (éluant AcOEt/hexane=1:1) permet d'isoler le composé attendu (0,24 g, 87 %).
Caractéristiques physiques :
*RMN $^1$H (acétone-d6) : 1,46 (m, 1H); 1,61 (m, 1H); 2,46-2,81 (m, 4H); 3,01 (dd, J=7,00-14,98 Hz, 1H); 4,05 (d, J=6,68 Hz, 1H); 6,54 (d, J=2,54 Hz, 1H); 6,66 (s, 1H); 6,68 (s, 1H); 6,71 (dd, J=2,54-8,34 Hz, 1H); 7,18 (d, J=8,34 Hz, 1H); 7,50 (s large, 2H); 7,83 (s large, 1H).
*SM (IE) : 268 (82), 251 (31), 240 (100), 161 (21), 145 (26), 77 (30).

Exemple 8 : **Préparation du 5,6,6a,7,8,12b-hexahydro-2,3,10-trihydroxy-benzo[c]phénanthrène (BXT01051)**

2'-(3,4-Diméthoxyphényl)tétral-6-méthoxy-2-spiro-1'-cyclopropan-1-one

A un mélange d'iodure de triméthylsulfoxonium (4,40 g, 20 mmol) et d'hydrure de sodium (à 80% dans l'huile minérale, 0,80 g, 20 mmol) sont ajoutés 10 ml de DMSO. Le mélange est agité à température ambiante pendant 1,5 h. On rajoute 30 ml de DMSO, puis on ajoute, par petites portions, la 2-(3,4-diméthoxybenzylidéne)-6-méthoxy-1-tétralone décrite précédemment, en 15 min. Le mélange est agité pendant 18 h à température ambiante et ensuite hydrolysé par l'addition de 50 ml d'une solution aqueuse saturée de $NH_4Cl$. On extrait le mélange avec de l'AcOEt. Les phases organiques rassemblées sont lavées à l'eau, séchées sur $MgSO_4$ et évaporées sous pression réduite, pour donner une huile incolore (7,0 g). La purification de cette huile par chromatographie, en éluant avec une mélange AcOEt/hexane (1:4), fournit le produit désiré (5,41 g, 85%).
Caractéristiques physiques :
*RMN 1H ($CDCl_3$) : 1,26 (dd, J=4,24-7,08 Hz, 1H); 1,68 (m, 1H); 1,85 (m, 2H); 2,70 (m, 2H); 2,94 (dd, J=7,08-8,24 Hz, 1H); 3,83 (s, 3H); 3,85 (s, 3H); 3,86 (s, 3H); 6,64 (d, J=2,52 hz, 1H); 6,77 (m, 3H); 6,82 (dd, J=2,52-8,50 Hz, 1H); 8,00 (d, J=8,72 Hz, 1H).
*SM (IE) : 338 (100), 323 (12), 200 (20), 176 (19), 151 (73), 91 (29), 77 (51).

2-(3,4-Diméthoxyohényléthyl)-6-méthoxy-1-tetralone

Une solurion du dérivé cyclopropanique précédemment obtenu (1,0 g, 2,96 mmol) et d'acide paratoluènesulfonique (300 mg) dans 20 ml de $CH_2Cl_2$ est agitée à température ambiante pendant 5 h. Cette solution est ensuite lavée avec une solution aqueuse saturée de $NaHCO_3$, séchée sur $MgSO_4$ et évaporée sous pression réduite pour donner une huile jaune (1,05 g). La purification de ce produit par chromatographie (éluant : AcOEt/hexane=1:4) donne un solide 10,57 g) qui est un mélange des ceux alcènes isomères.

0,53 g de ce dernier mélange est repris par 15 ml d'AcOEt. La solution est hydrogénée, en présence de palladium sur charbon (à 10%, 50 mg), sous une pression d'environ $2.10^5$ Pa (2 bars) d'hydrogène pendant 1 h. Le catalyseur est filtré et le filtrat est évaporé sous pression réduite, pour donner le produit attendu (0,53 g, 57%).
Caractéristiques physiques :

*RMN $^1H$ ($CDCl_3$) : 1,80 (m, 2H); 2,24 (m, 2H); 2,42 (m, 1H), 2,66 (m, 2H); 2,92 (m, 2H); 3,81 (s, 3H); 3,82 (s, 3H); 3,84 (s, 3H); 6,64 (d, J=2,36 Hz, 1H); 6,77 (m, 3H); 6,79 (dd, J=2,36-8,80 Hz, 1H); 7,97 (d, J=8,72 Hz, 1H).
*SM (IE) : 340 (2,5), 176 (100), 164 (16), 151 (12).

5,6,7,8-Tétrahydro-2,3,10-triméthoxy-benzo[c]phénanthrène.

8 g d'acide polyphosphorique sont mélangés avec la tétralone obtenue précédemment (0,41 g, 1,21 mmol), puis chauffés à 80-90°C pendant 2,5 h. Après avoir refroidi le mélange à température ambiante, 20 g d'eau glacée et 20 ml d'AcOEt sont ajoutés. On décante la phase organique et extrait la phase aqueuse avec de l'AcOEt. Les phases organiques rassemblées sont lavées avec une solution aqueuse saturée de $NaHCO_3$ puis avec une solution aqueuse saturée de NaCl, séchées sur $MgSO_4$ et évaporées à sec pour donner un solide (0,38g) qui est purifié par chromatographie en éluant avec un mélange AcOEt/hexane (1:9). On obtient le produit attendu (0,19 g; 49%).
Caractéristiques physiques :
*RMN $^1H$ ($CDCl_3$): 2,31 (m, 4H); 2,66 (m, 4H); 3,80 (s, 3H); 3,82 (s, 3H); 3,89 (s, 3H); 6,77 (m, 3H); 7,08 (s, 1H); 7,44 (d, J=8,42 Hz, 1H).
*SM (IE) : 322 (100), 307 (10), 291 (19), 189 (18), 161 (18).

5,6,6a,7,8,12b-Hexahydro-2,3,10-triméthoxy-benzo[c]phénanthrène

A une solution au précédent produit (170 mg, 0,50 mmol) dans 10 ml d'AcOEt, sont ajoutés 20 mg de palladium sur charbon (à 10%). Le mélange est hydrogéné sous une pression d'environ $8.10^5$ Pa (8 atm) pendant 2,5 h. Le catalyseur est éliminé par filtration et le filtrat est évaporé à sec pour donner une huile. Cette huile est purifiée par chromatographie, en éluant avec un mélange AcOEt/hexane (1:9), pour donner le produit désiré (150 mg, 88%).
Caractéristiques physiques :
*RMN $^1H$ ($CDCl_3$): 1,53 (m, 2H); 1,81 (m, 1H); 2,00 (m, 1H); 2.32 (m, 1H); 2,75 (m, 4H); 3,77 (s, 3H); 3,78 (s, 3H); 3,81 (1H, caché par les OMe); 3,85 (s, 3H); 6,65 (m, 4H); 6,99 (d, J=9,16 Hz, 1H).
*SM (IE): 324 (100), 309 (5), 296 (33), 293 (37), 203 (36), 189 (86), 173 (39).

5,6,6a,7,8,12b-Hexahydro-2,3,10-trihydroxy-benzo[c]phénanthrène

A une solution du dérivé précédemment obtenu (150 mg, 0,37 mmol) dans 1 ml de $CH_2Cl_2$, refroidie à -70°C, est ajoutée goutte à goutte une solution de $BBr_3$ dans $CH_2Cl_2$ (1,0 M, 1,5 ml, 1,5 mmol). La solution rouge obtenue est réchauffée à température ambiante et agitée pendant 0,5 h à cette température. Le mélange réactionnel est versé dans un mélange d'eau glacée et d'AcOEt. La phase aqueuse est saturée par NaCl, puis extraite deux fois à l'AcOEt. Les pnases organiques rassemblées sont lavées avec une solution aqueuse saturée de NaCl, séchées sur $MgSO_4$ et évaporées à sec, pour donner une huile jaune (0,18 g). Une chromatographie de cette huile (éluant : AcOEt/hexane=1:1) permet d'isoler le composé attendu (91 mg, 87%).
Caractéristiques physiques :
*RMN $^1H$ (acétone -d6): 1,53 (m, 2H); 1, 90 (m, 2H); 2,32 (m, 1H); 2,75 (m, 4H); 3,67 (d, J=4,76 Hz, 1H): 6,48 (s, 1H); 6,55 (s, 1H), 6,59 (m, 2H); 6,88 (d, J=8,88 Hz, 1H); 7,51 (s large, 2H); 7,95 (s large, 1H).
*SM (IE) : 282 (100), 265 (16), 254 (53), 175 (26), 161 (36), 115 (31), 91 (22), 77 (24).

D. Composés de la série IV :

Exemple 9 : **Préparation de la 3-O-méthylbraziline**

5',6',7-Tri-O-benzylbraziline :

Une solution de braziline monohydratée (304 mg; 1,0 mmol; ALDRICH) dans de l'acétone (2,5 ml) contenant du bromure de benzyle (0,4 ml; 3,36 mmol; JANSSEN) et du carbonate de potassium anhydre (1,06 g; 7,68 mmol; OSI), sous atmosphère inerte, est chauffée à reflux pendant 15 h. Le mélange réactionnel est de couleur violette. Une solution de soude (5 ml, 0,5 N) est ajoutée, et la solution ainsi obtenue est agitée pendant 1 h à température ambiante. Le milieu réactionnel est extrait par de l'acétate d'éthyle (2x10 ml). Les phases organiques sont rassemblées, lavées à l'eau (2x5 ml) et séchées sur $MgSO_4$. Le solvant est évaporé sous pression réduite. Le produit désiré est purifié par chromatographie sur colonne de silice MERCK F254 (éluant = hexane/AcOEt, 3:1).
Rendement = 530 mg (95,3 %).
Caractéristiques physiques :
* RMN $^1H$ (200 MHz, $CDCl_3$) :

2,45 (s, 1H); 2,83 (d, 1H, J=16,0 Hz); 3,18 (d, 1H, J=16,0 Hz; 3,78 (d, 1H, J=11,3 Hz); 4,02 (d, 1H, J=11,3 Hz); 4,09 (s, 1H); 5,04 (s, 2H); 5,09 (s, 2H); 5,11 (s, 2H); 6,55 (d, 1H, J=2,6 Hz); 6,71 (dd, 1H, J=2,6-8,5 Hz); 6,80 (s,1H); 6,88 (s, 1H); 7,17 (d, 1H, J=8,5 Hz); 7,42 (m, 15H)
* SM (IE) :($M^+$) = 556(2), 466(1), 181(2), 108(3), 91(100).

5',6',7-Tri-O-benzyl-3-O-méthylbraziline :

Dans une solution de 5',6',7-tri-O-benzyl-braziline (530 mg; 0,95 mmol) dans du DMSO sec (4 ml), on introduit de la potasse en poudre (210 mg; 3,80 mmol), sous atmosphère inerte, à température ambiante. De l'iodure de méthyle (0,12 ml; 1,90 mmol) est alors ajouté au milieu réactionnel qui est agité 30 minutes dans ces conditions. Après addition d'eau (5 ml), la solution est extraite avec de l'acétate d'éthyle (15 ml). La phase organique est séchée sur $MgSO_4$. Le solvant est évaporé sous pression réduite. Le produit désiré est purifié par chromatographie sur colonne de silice MERCK F254 (éluant = hexane/AcoEt, 2:1).
Rendement = 510 mg (94 %).

Le produit est recristallisé dans un mélange hexane/AcOEt 12:1) .
Caractéristiques physiques :
* pF = 99,5-100,5°C.
* RMN $^1$H (200 MHz, $CDCl_3$) :
2,70 (d, 1H, J=15,8 Hz); 3,22 (d, 1H, J=15,8 Hz); 3,38 (s, 3H); 3,67 (d, 1H, J=11,9 Hz); 4,20 (s, 1H); 4,26 (d, 1H, J=11,9 Hz), 5,03 (s, 2H); 5,07 (s, 2H); 5,11 (s, 2H); 6,54 (d, 1H, J=2,6 Hz); 6,68 (dd, 1H, J=2,6-8,5 Hz); 6,78 (s,1H); 6,88 (s, 1H); 7,17 (d, 1H, J=8,5 Hz); 7,42 (m, 15H).
* SM (IE) : ($M^+$) = 570(45), ($M^+$-91) = 479(12), 91(100).

3-O-Méthylbraziline :

Une solution de 5',6',7-tri-O-benzyl-3-O-méthylbraziline (160 mg; 0,28 mmol) dans un mélange AcOEt/MeOH (5:2) contenant du palladium à 10 % sur charbon (20 mg) est purgée pendant 15 minutes par un courant d'hydrogène. La suspension est hydrogénée pendant 5 h à température ambiante, sous agitation. Elle est ensuite filtrée sous atmosphère inerte. Le solvant est évaporé sous pression réduite. Le produit désiré cristallise progressivement. Rendement = 81 mg (96 %).
Caractéristiques physiques :
* RMN $^1$H (200 MHz, $CD_3COCD_3$) :
2,68 (d, 1H, J=15,6 Hz); 3,08 (d, $^1$H, J=15,6 Hz); 3,28 (s, 3H); 3,52 (d, 1H, J=12,1 Hz); 4,03 (s, 1H); 4,24 (d, 1H, J=12,1 Hz); 6,28 (d, 1H, J=2,5 Hz); 6,48 (dd, 1H, J=2,5-8,5 Hz); 6,64 (s, 1H); 6,71 (s, 1H); 7,18 (d, 1H, J=8,5 Hz); 7,79 (s, 1H); 7,85 (s, 1H); 8,43 (s, 1H).
* SM (IE): 300(5), 268(4), 110(6), 84(41), 66(49).

Exemple 10 : **Préparation du brazilane ($R^1$ = OH ; $R^2$ = $-CH_2-O-$ ; $R^3$ = H).**

5',6',7-Tri-O-benzyl-3-O-phényloxythiono-carbonylbraziline :

A une solution de 5',6',7-tri-O-benzylbraziline (280 mg, 0,50 mmol) dans 3 mi de THF, refroidie à -78°C, est ajoutée goutte à goutte, sous azote et sous agitation, une solution de méthyllithium (0,4 ml d'une solution 1,6 M dans le cyclohexane, JANSSEN). L'agitation est maintenue 10 minutes à cette température, puis 1 heure à température ambiante. Le mélange réactionnel est de nouveau refroidi à -78°C et du chlorothionocarbonate de phényle (0,21 ml, 0,75 mmol, ALDRICH) est ajouté goutte à goutte. On laisse le mélange réactionnel revenir à la température ambiante et on l'agite pendant 1 heure. L'hydrolyse est réalisée à l'aide de chlorure d'ammonium puis le produit est extrait par de l'acétate D'éthyle (2x10 ml). Les phases organiques sont rassemblées, lavées avec une solution saturée en chlorure de sodium (2x10 ml), puis séchées sur $MgSO_4$. Le solvant est évaporé sous vide. L'huile jaune ainsi obtenue est chromatographiée sur colonne de silice MERCK F254. Le produit désiré, élué par un mélange éther/hexane (1:5), est obtenu avec un rendement de 83 % (280 mg d'un solide incolore).
Caractéristiques physiques :
*RMN $^1$H (200 MHz, $CDCl_3$) :
3,51 (d, 1H, J =16,50 Hz); 3,71 (d, 1H, J = 16,50 Hz); 3,75 (d, 1H, J = 12,17 Hz); 4,58 (s, 1H); 5,05 (s, 4H); 5,09 (s, 2H); 5,27 (d, 1H, J = 12,17 Hz); 6,57 (d, 1H, J = 2,56 Hz); 6,69 (dd, 1H, J = 2,56-8,45 Hz); 6,78 (s, 1H); 6,88 (s, 1H); 7,06 (d, 2H, J = 7,30 Hz); 7,19 (d, 1H, J = 8,45 Hz); 7,24-7,46 (m, 18H).
* SM (IE) : ($M^+$-PhOC(OH)=S)=538(3); 447(1,5); 419(3); 91(100); 60(80).

5',6',7-Tri-O-benzylanhydrobraziline

Une solution du dérivé thionocarbonate précédemment obtenu (100 mg, 0,15 mmol) dans 3 ml de toluène sec est chauffée au reflux pendant 2 heures. Le solvant de la solution légèrement jaunâtre, ainsi obtenue, est évaporé sous vide. Le résidu est purifié par chromatographie sur colonne de silice MERCK F254 et élué par un mélange éther/hexane (1:5). Le produit désiré est obtenu avec un rendement de 82 % (64 mg).
Caractéristiques physiques :
* pF = 142-144°C (acétate d'éthyle/hexane=1:4).
* RMN 1H (200 MHz, $CDCl_3$) :
3,30 (s, 2H); 5,05 (s, 2H); 5,12 (s, 2H); 5,17 (s, 2H); 5,21 (s, 2H); 6,58 (s, 1H); 6,60 (dd, 1H, J = 2,50-8,41 Hz); 7,13 (s, 1H)) 7,30-7,50 (m, 17H).
* SM (IE) : 538(12), 447(3), 419(6), 91(100).

Brazilane :

Une solution du dérivé précédent (92 mg, 0,17 mmol) dans 5 ml d'acétate d'éthyle est hydrogénée en présence de palladium sur charbon (à 10 %, 30 mg, ALDRICH) sous une pression d'environ $8.10^5$ Pa (8 bars) pendant 3 heures à température ambiante. La suspension est filtrée et le solvant est évaporé sous vide. Le produit désiré est obtenu après purification par chromatographie sur colonne de silice MERCK F254 avec un éluant éther/hexane (1:1). Rendement = 69 %.
Caractéristiques physiques :
* RMN $^1H$ (200 MHz, $CD_3COCD_3$) :
2,47 (dd, 1H, J = 2,38-15,46 Hz); 2,80 (m, 1H); 3,03 (dd, 1H, J = 7,32-15,46 Hz); 3,47 (t, 1H, J = 10,62 Hz); 4,02 (dd, 1H, J = 4,48-10,62 Hz); 4,06 (d, 1H, J = 7,34 Hz); 6,25 (d, 1H, J = 2,52 Hz); 6,45 (dd, 1H, J = 2,52-8,34 Hz); 6,67 (s, 1H); 6,80 (s, 1H); 7,20 (d, 1H, J = 8,34 Hz); 7,55 (s, 1H); 7,61 (s, 1H); 8,15 (s, 1H).
* SM (IE) : 270(100), 253(31), 240(18), 205(22), 161(39), 115(25), 101(24).

E. Composé de la série V :

Exemple 11 **: Préparation de la 7-O-méthylbraziline**

5',6'-O-Méthylènebraziline :

Une solution de fluorure de potassium anhydre (145 mg, 2,5 mmol, JANSSEN) et de monohydrate de braziline (152 mg, 0,5 mmol, ALDRICH) dans 1 ml de diméthylformamide est chauffée et agitée sous azote à 120°C. Du dibromométhane (39 µl, 0,55 mmol, JANSSEN) est ajouté. Après 18 heures d'agitation à cette température, le mélange réactionnel est refroidi à 0°C et 4 ml d'eau sont ajoutés. Le mélange est extrait avec de l'acétate d'éthyle (2x5 ml). Les phases organiques sont rassemblées, lavées avec de l'eau puis avec une solution aqueuse saturée de chlorure de sodium et séchées sur $Na_2SO_4$. Le solvant est évaporé sous vide. Le produit désiré est obtenu après purification par chromatographie sur colonne de silice MERCK F254 (éluant = acétate d'éthyle/hexane, 1:1).
Rendement : 42 %.

Le produit est recristallisé dans un mélange acétate d'éthyle/hexane (1:1).
Caractéristiques physiques :
* pF = 264,5°C (décomposition).
* RMN $^1H$ (200 MHz, DMSO $d_6$) :
2,81 (d, 1H, J = 16,3 Hz); 2,86 (d, 1H, J = 16,3 Hz); 3,64 (d, 1H, J = 11,8 Hz); 3,82 (d, 1H, J = 11,8 Hz); 3,88 (s, 1H; 5,36 (s, 1H) ; 5,91 (s, 1H); 5,93 (s, 1H); 6,18 (d, 1H, J = 2,4 Hz); 6,40 (dd, 1H, J = 2,4-8,2 Hz); 6,74 (s, 1H); 6,87 (s, 1H); 7,22 (d, 1H, J = 8,20 Hz); 9,26 (s, 1H)
* SM (IE) : 298(100), 280(62), 279(79), 241(28), 149(58).

7-O-Méthyl-5',6'-O-méthylénebraziline:

A une solution de 5',6'-O-méthylènebraziline (79 mg, 0,27 mmol) dans 2 ml d'acétone, sont ajoutés du carbonate de potassium (183 mg, 1,33 mmol, OSI) et de l'iodure de méthyle (35 µl, 0,53 mmol, LANCASTER). Le mélange est chauffé au reflux sous azote pendant 24 heures sous agitation. Puis, après retour à température ambiante, la suspension est filtrée. Le filtrat est évaporé à sec et le produit désiré est obtenu après purification par chromatographie sur colonne de silice MERCK F254 (éluant = acétate d'éthyle/hexane, 1:4). Rendement = 65 %.
Caractéristiques physiques :

* RMN $^1H$ (200 MHz, $CDCl_3$) :
2,58 (s, 1H); 2,80 (d, 1H, J = 16,45 Hz); 3,17 (d, 1H, J = 16,45 Hz); 3,76 (s, 3H); 3,79 (d, 1H, J = 11,8 Hz); 4,00 (d, 1H, J = 16,45 Hz); 4,04 (s, 1H); 5,86 (m, 2H); 6,48 (d, 1H, J = 2,4 Hz); 6,62 (dd, 1H, J = 2,4-8,2 Hz); 6,68 (s, 1H); 6,72 (s, 1H); 7,24 (d, 1H, J = 8,20 Hz).

7-0-Méthylbraziline :

A une solution de 7-O-méthyl-5',6'-O-méthylène-braziline (50 mg, 0,16 mmol) dans 2 ml de chlorure de méthylène, est ajoutée goutte à goutte, sous azote et agitation, une solution de trichlorure de bore (0,25 ml d'une solution 1M dans le chlorure de méthylène, 0,25 mmol). Le mélange réactionnel est agité à température ambiante pendant 20 heures. Du méthanol absolu (60 µl) est ajouté lentement puis le solvant est évaporé sous vide. Le résidu est repris par de l'acétate d'éthyle (5 ml) et lavé à l'eau. La phase organique est séchée sur $Na_2SO_4$ et le solvant est évaporé sous vide. Le produit désiré est obtenu après purification par chromatographie sur colonne de silice MERCK F254 (éluant = acétate d'éthyle/hexane, 1:1). Rendement = 44 %.
Caractéristiques physiques
* RMN $^1H$ (200 MHz, $CD_3COCD_3$) :
2,80 (d, 1H, J = 16,5 Hz); 3,00 (d, 1H, J = 16,50 Hz); 3,70 (s et d, 4H); 3,93 (d, 1H, J = 11,30 Hz); 3,98 (s, 1H); 4,48 (s, 1H); 6,37 (d, 1H, J = 2,4 Hz); 6,56 (dd, 1H, J = 2,4-8,30 Hz) ; 6,63 (s, 1H); 6,77 (s, 1H); 7,26 (d, 1H, J = 8,30 Hz); 7,59 (s, 1H); 7,64 (s, 1H).
* SM (IE) : 300(100), 282(59), 281(63), 243(45), 176(13), 165(10), 141(20).

II Préparation de composés de formule générale II ("réactifs piégeurs de mercaptans") :

Exemple 12 **: Préparation du tétrafluoroborate de 1,4,6-triméthyl-2-vinylpyridinium**

4,6-Diméthyl-2-vinylpyridine :

A une solution de 2,4-diméthylpydirine (20 g; 0,186 mol; LANCASTER) dans 200 ml de THF fraîchement distillé, refroidie à -20°C et sous atmosphère inerte, on ajoute du chlorure de benzoyle (29, 12 g; 0,186 mol; JANSSEN) dans 100 ml de THF. Le mélange réactionnel est agité à cette température pendant 20 minutes. Puis du bromure de vinylmagnésium (186 ml d'une solution 1,0 M dans le THF) est ajouté et la solution est agitée à température ambiante pendant 0,5 h. Le solvant est évaporé sous pression réduite et le résidu est repris par 300 ml d'éther terbutylméthylique. Après lavage du milieu réactionnel avec une solution saturée de $NH_4Cl$, la phase organique est séchée sur $MgSO_4$ et le produit désiré est purifié par chromatographie sur colonne de silice MERCK F254 (éluant = hexane/AcOEt, 9:1). Rendement = 39,5 g (83 %), huile incolore.

Le produit précédent est repris par 500 ml de toluène et additionné de p-chloranil (tétrachloro-1,4-benzoquinone) (41,7 g; 0,169 mol), puis chauffé à reflux pendant 2 h. Le milieu réactionnel est concentré sous pression réduite, puis additionné d'une solution de soude (260 ml, 2N). Le produit est extrait par de l'éther terbutylméthylique (300 ml) et purifié par chromatographie sur colonne de silice MERCK F254 (éluant = hexane/AcOEt, 9:1).
Rendement = 11,1 g (54,2 %), huile jaune pâle.
Caractéristiques physiques :
* RMN $^1H$ (200 MHz, $CD_3COCD_3$) :
2,20 (s, 3H); 2,50 (s, 3H); 5,25-5,45 (dd, 2H, J = 11,9-18,7 Hz); 6,60-6,90 (m, 3H).
* SM (IC, $NH_3$) : $MH^+$ = 134(100).

Tétrafluoroborate de 1,4,6-triméthyl-2-vinylpyridinium :

A une solution de 4,6-diméthyl-2-vinylpyridine (0,61 g; $4,13.10^{-3}$ mol) dans 4,0 ml de chlorure de méthylène sous atmosphère inerte on ajoute, en une portion, du tétrafluoroborate de triméthyloxonium (0,50 g; $3,76.10^{-3}$ mol). La suspension est laissée réagir pendant 2 h à 25°C. La suspension est filtrée et le produit est séché sous vide. Rendement = 0,162 g (18 %), cristaux blancs.
Caractéristiques physiques :
* RMN $^1H$ (200 MHz, $CD_3COCD_3$) :
2,60 (s, 3H); 2,85 (s, 3H); 4,20 (s, 3H); 6,00 (d, 1H, J=12,8 Hz); 6,25 (d, 1H, J=19,2 Hz); 7,27 (dd, 1H, J=12,8-19,2 Hz); 7,82 (s, 1H); 7,95 (s, 1H).
* SM (IE) : ($M^+$) 148(10); 147(18); 133(34); 121(100); 106(25); 91(31); 77(52); 49(77).
* Microanalyse :
Calculé (%) : C 51,10; H 6,00; N 6,00

Trouvé (%) : C 49,80; H 6,08; N 5,96.

Exemple 13 : **Préparation du tétrafluoroborate de 1-méthyl-4-vinylquinoléinium**

4-Vinylquinoléine :

Une solution de 4-quinoléinecarboxaldéhyde (5 g; $3{,}18.10^{-2}$ mol) dans 32 ml de benzène, sous atmosphère inerte, contenant de l'iodure de triphénylméthylphosphonium (26 g; $6{,}36.10^{-2}$ mol) est ajoutée à une solution de soude (96 ml; 5N). Le système biphasique est agité à 45°C pendant 30 minutes. Après décantation, la phase aqueuse est extraite par de l'acétate d'éthyle (3x100 ml). Les phases organiques sont réunies et lavées par une solution d'HCl 1N (2x30 ml), puis la phase aqueuse est neutralisée par du $NaHCO_3$ et extraite par du chlorure de méthylène (3x100 ml). La phase organique est séchée sur $MgSO_4$. Le produit désiré est purifié par chromatographie sur colonne de silice MERCK F254 (éluant = cyclohexane/AcOEt, 8:2).
Rendement = 2,8 g (60 %).
Caractéristiques physiques :
* RMN $^1H$ (200 MHz, $CDCl_3$) :
5,60 (d, 1H, J=10 Hz); 5,95 (d, 1H, J=14 Hz);7,30-7,55 (m, 4H); 7,60-7,75 (t, 1H, J=4 Hz);8,07 (t, 2H, J=6 Hz); 8,85 (d, 1H, J=4 Hz).

Tétrafluoroborane de 1-méthyl-4-vinylquinoléinium :

A une solution de 4-vinylquinoléine (0,72 g; $4{,}64.10^{-3}$ mol) dans un mélange acétonitrile/acétone (2,5:1), est ajouté sous atmosphère inerte, en une portion, du tétrafluoroborate de triméthyloxonium (1,58 g; $10{,}7.10^{-3}$ mol). La suspension est laissée réagir pendant 1 h à 25°C. Le solvant est évaporé sous pression réduite, le résidu est repris par du 2-propanol (25 ml) puis agité pendant 30 minutes à température ambiante. Après addition d'éther éthylique (25 ml), la suspension est filtrée. Le produit désiré est séché sous vide.
Rendement = 0,93 g (60,4 %).
Caractéristiques physiques :
* RMN $^1H$ (200 MHz, $CD_3COCD_3$) :
4,30 (s, 3H); 6,25 (d, 1H, J=12,5 Hz); 6,65 (d, 1H, J=17,5 Hz) ; 7,85-8,00 (dd, 1H, J=12,5-17,5 Hz); 8,10 (d, 1H, J=7 Hz); 8,35 (m, 2H); 8,57 (d, 1H, J=10 Hz); 8,72 (d, 1H, J=10 Hz); 9,35 (d, 1H, J=7 Hz).

* SM (FAB) glycérol-NOBA : ion positif $M^+$ = 170(100)
ion négatif $M^-$ = 87(100).

FAB = bombardement par atomes rapides (Fast Atom Bombardment).
NOBA = alcool méta-nitrobenzylique.
* Microanalyse :
Calculé (%) : C 56,00; H 4,60; N 5,40.
Trouvé (%) : C 55,56; H 4,35; N 4,12.

III. Exemples d'applications :

Les protocoles opératoires décrits ci-après sont des exemples d'application du procédé de dosage utilisant respectivement les réactifs BXT01041 (décrit à l'exemple 1), BXT01048 (décrit à l'exemple 3), BXT01049 (décrit à l'exemple 4) et BXT01050 (décrit à l'exemple 7) la braziline (produit commercial), le brazilane (composé de l'exemple 10) et la 3-O-méthylbraziline (composé de l'exemple 9), pour mesurer l'activité SOD dans un lysat érythrocytaire.

Exemple 14 **: Procédure de dosage utilisant le réactif BXT01041**

On utilise un coffret contenant les réactifs décrits ci-dessous. Ce coffret de réactifs doit être stocké à une température comprise entre 0 et 4°C.

Description des réactifs :

**R1** : Solution de BXT01041 $2.10^{-3}$ M dans le mélange DMSO/$H_2O$, 50/50 (v/v) contenant de l'acide borique 0,15 M. (A conserver entre 0 et 4°C pendant les mesures; stable au moins 1 mois après préparation, dans les conditions de stockage mentionnées ci-dessus).

**R2** : Solution de tétrafluoroborate de 1,4,6-triméthyl-2-vinylpyridinium $5.10^{-2}$ M dans du DMSO contenant 25 % (p/v) d'éthylène-glycol.
(A conserver entre 0 et 4°C pendant les mesures; stable au moins 1 mois après préparation).

**E** : Ethanol absolu/chloroforme, 62,5/37,5 (v/v).
(A conserver entre 0 et 4°C pour la préparation de l'échantillon).

**T** : Tampon AMPD 0,05 M/HCl pH = 8,8, contenant du DTPA 0,1 mM.
(Laisser équilibrer à l'air et à 37°C avant les mesures).

Préparation de l'échantillon :

On centrifuge au moins 100 µl de sang total à 4°C et 3000 tr/min pendant 10 minutes. On élimine le surnageant. On lave le culot érythrocytaire avec une solution de chlorure de sodium à 0,9 % (refroidie entre 0 et 4°C) puis on centrifuge à 4°C et 3000 tr/min pendant 10 minutes. On élimine le surnageant. On reprend le culot érythrocytaire avec 4 fois son volume d'eau distillée refroidie entre 0 et 4°C et on mélange.

A 125 µl de ce lysat érythrocytaire au 1/5ème, d'une part, et à 125 µl d'eau distillée, d'autre part on ajoute 200 µl du mélange **E** (refroidi entre 0 et 4°C). On mélange bien chacune des solutions et on centrifuge à 4°C et 3000 tr/min pendant 10 minutes. Le surnageant ainsi obtenu servira au dosage, respectivement pour l'échantillon (débarrassé de l'hémoglobine) et pour le témoin.

Les surnageants doivent être conservés entre 0 et 4°C pour les mesures.

Procédure de dosage :

Les mesures sont effectuées à 37°C dans un spectrophotomètre dont les cuves de mesure sont thermostatées.

Le mélange réactionnel correspondant à chaque dosage est préparé extemporanément selon les proportions données dans le tableau ci-dessous pour des cuves de 1 ml.

Chaque réaction est déclenchée par l'ajout de 10 µl de réactif **R1**. Après homogénéisation du milieu, l'évolution de l'absorbance est enregistrée immédiatement et suivie pendant 1 min 30 s.

La mesure d'absorbance est effectuée à 501 nm dans des cuves de 1 cm de trajet optique, contre de l'air.

Pour cnaque mesure, le milieu réactionnel défini dans le tableau ci-dessous doit être incubé pendant 1 minute à 37°C avant ajout du réactif **R1**.

| | **TEMOIN** | **ECHANTILLONS** |
|---|---|---|
| **R2** | 20 µl | 20 µl |
| **Blanc échantillon (*)** | 40 µl | - |
| **ECHANTILLONS** | - | 40 µl |
| **T** | 930 µl | 930 µl |

(*) : Eau distillée ayant subi la même procédure d'extraction que le lysat érythrocytaire.

Pour chacune des mesures, la vitesse maximale d'autoxydation, exprimée en unités d'absorbance par minute, est déterminée en évaluant la pente du décours temporel d'absorbance (vers le temps t=30s). Cette pente correspond à une phase d'autoxydation du réactif BXT01041 qui doit être linéaire.

Expression des résultats :

Soient $V_c$ et $V_s$ les vitesses correspondant respectivement au témoin et à l'échantillon, l'activité SOD de celui-ci est calculée à l'aide de l'équation de la droite d'étalonnage présentée sur la figure 1 qui représente les variations de l'inverse de la différence ($V_s$-$V_c$), exprimé en min/variation de l'absorbance (min/Δ abs.), en fonction de l'inverse de la concentration en SOD, exprimé en ml/U. Cette droite permet de déterminer la valeur de l'abscisse correspondant à une valeur d'ordonnée expérimentalement mesurée.

On multiplie ensuite l'inverse de cette abscisse par le facteur de dilution D.

Les résultats sont alors exprimés en unités d'activité SOD par ml d'échantillon, selon :

$$\text{Unités d'activité SOD} = 1/\{\ 0{,}026 \times [1/(V_S - V_C)] - 0{,}075\ \} \times D$$

L'unité U d'activité SOD définie dans cette méthode présente l'avantage unique de fournir une référence invariable,

compte tenu de l'absence de réactifs protéiques ou enzymatiques peu stables, tels que la xanthine oxydase.

L'utilisation de cette unité est fortement recommandée, mais l'utilisateur peut la traduire en valeur pondérale d'une SOD purifiée avec laquelle il aura préalablement construit une courbe d'étalonnage. Une telle estimation pondérale sera fortement affectée par le pourcentage d'enzyme inactive présent dans la préparation.

Les résultats peuvent aussi être exprimés, par exemple, en U/ml de sang total ou en U/mg de protéines érythrocytaires.

Remarques :

Le contrôle de la procédure de dosage peut être effectué en dosant l'activité SOD d'un lysat érythrocytaire préparé comme décrit précédemment et conservé à -70°C sous forme d'aliquots pour servir de témoin. L'activité SOD de tels aliquots est stable au moins 6 mois à -70°C.

Le dosage doit être effectué sur un aliquot décongelé extemporanément.

Exemple 15 : **Procédure de dosage utilisant le réactif BXT01048**

Identique à l'exemple 14, excepté que :

- Dans **R1**, le réactif BXT01041 est remplacé par le réactif BXT01048.
- L'évolution de l'absorbance est suivie à 505 nm.

La figure 2 annexée représente les variations de l'inverse de la différence (Vs-Vc), exprimé en min/variation de l'absorbance (min/Δabs.) en fonction de l'inverse de la concentration en SOD, exprimé en ml/U, dans cet exemple.

$$\text{Unités d'activité SOD} = 1/\ \{0{,}028 \times [1/(V_s - V_c)] - 0{,}077\ \} \times D$$

Exemple 16 : **Procédure de dosage utilisant le réactif BXT01049**

Identique à l'exemple 14, excepté que :

- **R1** est remplacé par $\mathbf{R_3}$ qui est défini de la façon suivante :

  solution de BXT01049 $2.10^{-3}$M dans le mélange DMSO/$H_2O$ à 50/50 (v/v), contenant de l'acide borique 0,1M.

- L'évolution de l'absorbance est suivie à 522 nm pendant 1 min.

La figure 3 annexée représente les variations de l'inverse de la différence (Vs-Vc), exprimé en min/variation de l'absorbance (min/Δabs.) en fonction de l'inverse de la concentration en SOD, exprimé en ml/U, dans cet exemple.

$$\text{Unités d'activité SOD} = 1/\{0{,}023 \times [1/(V_s - V_c)] - 0{,}063\} \times D.$$

Exemple 17 : **Procédure de dosage utilisant le réactif BXT01050**

Identique à l'exemple 14, excepté que :

- **R1** est remplacé par **R4** qui est défini de la façon suivante :

  solution de BXT01050 $2.10^{-3}$ M dans le mélange DMSO/$H_2O$ à 50/50 (v/v), contenant de l'acide borique 0,3M.

- L'évolution de l'absorbance est suivie à 525 nm pendant 1 min.

La figure 4 annexée représente les variations de l'inverse de la différence (Vs-Vc), exprimé en min/variation de l'absorbance (min/Δabs.) en fonction de l'inverse de la concentration en SOD, exprimé en ml/U, dans cet exemple.

$$\text{Unités d'activité SOD} = 1/\{0{,}070 \times [1/(Vs\text{-}Vc)] - 0{,}102\} \times D.$$

Exemple 18 : **Procédure de dosage utilisant la braziline.**

On utilise un coffret identique à celui utilisé dans l'exemple 14, si ce n'est que dans **R1** le réactif BXT 01041 est remplacé par de la braziline.

Le lysat érythrocytaire est obtenu comme décrit dans l'exemple 14.

A 125 µl de ce lysat érythrocytaire au 1/5ème, on ajoute 200 µl du mélange **E**, (refroidi entre 0 et 4°C). On mélange et centrifuge à 4°C et 3000 tr/min pendant 10 minutes. Le surnageant ainsi obtenu, débarrassé de l'hémoglobine, servira au dosage (il doit être conservé à 4°C pour les mesures).

La procédure de dosage est la même que dans l'exemple 14, si ce n'est que la mesure d'absorbance est effectuée à 539 nm au lieu de 501 nm et que le "blanc échantillon" est remplacé par de l'éthanol à 50 % (v/v) dans de l'eau distillée.

Expression des résultats :

Dans les conditions de ce dosage, une unité d'activité SOD (U) correspond à une augmentation de la formation de braziléine (produit d'autoxydation de la braziline), par rapport au témoin, de 0,5 µmole.$1^{-1}$.$min^{-1}$.

La figure 5 annexée représente les variations de l'inverse de la différence ($V_s$-$V_c$), exprimée en min/variation de l'absorbance (min/Δ abs.) en fonction de l'inverse de la concentration en SOD, exprimé en ml/U, dans cet exemple.

$$\text{Unités d'activité SOD} = 1/\{0,034 \times [1/(V_s-V_c)] - 0,065\} \times D.$$

Exemple 19 : **Procédure de dosage utilisant le brazilane**

Le coffret de réactifs utilisé contient les mêmes réactifs que celui de l'exemple 14, à ceci près que le réactif BXT01041 est remplacé par le brazilane.

Préparation de l'échantillon :

Comme dans l'exemple 18.

Procedure de dosage :

On procède comme dans l'exemple 18, si ce n'est que chaque réaction est déclenchée par l'ajout de 10 µl de réactif **R1** contenant du brazilane au lieu de la braziline et que l'évolution de l'absorbance est suivie tendant 1 min à 520 nm au lieu de 1 min 30 s à 539 nm.

Expression des résultats :

La figure 6 annexée représente les variations de l'inverse de la différence ($V_s$-$V_c$) exprimé en min/variation de l'absorbance (min/Δabs.) en fonction de l'inverse de la concentration en SOD, exprimé en ml/U, dans cet exemple.

$$\text{Unités d'activité SDO} = 1/\{0,069 \times [1/V_s-V_c)] - 0,077\} \times D.$$

Exemple 20 : **Procédure de dosage utilisant la 3-O-méthylbraziline**

Le coffret de réactifs utilisé contient les mêmes réactifs que dans l'exemple 14, à ceci près que le réactif BXT01041 est remplacé par la 3-O-méthylbraziline.

Préparation de l'échantillon :

La préparation de l'échantillon est identique à celle décrite dans l'exemple 18.

Procédure de dosage :

Les mesures d'absorbance sont effectuées à 541 nm. A cette exception près, la procédure de dosage est identique à celle décrite dans l'exemple 18.

Expression des résultats :

L'expression des résultats est identique à celle décrite dans l'exemple 18 à l'aide d'une courbe d'étalonnage spécifique de la 3-O-méthylbraziline.

EXEMPLE 21 : **Autre procédure de dosage utilisant le réactif BXT01050**

On utilise un coffret contenant les réactifs décrits ci-dessous. Ce coffret doit être stocké à une température comprise entre 0 et 4°C.

Description des réactifs :

**S1** : Solution de BXT01050 $0{,}66.10^{-3}$ M dans HCl $3{,}2.10^{-2}$ M contenant du DTPA 0,5 mM et 2,5 % d'éthanol absolu. (A conserver entre 0 et 4°C pendant les mesures ; stable au moins 1 mois après préparation, dans les conditions de stockage mentionnées ci-dessus).

**S2** : Solution de trifluorométhylsulfonate ou de tétrafluoroborate de 1,4,6-triméthyl-2-vinylpyridinium $3{,}33.10^{-2}$ M dans le DMSO contenant 25 % (p/v) d'éthylène glycol.
(A conserver entre 0 et 4°C pendant les mesures ; stable au moins 1 mois après préparation).

**S3** : Tampon AMPD 0,055 M/HCl pH = 8,8 (à 37°C) contenant de l'acide borique 3,33 mM et du DTPA 0,11 mM. (Laisser équilibrer à l'air et à 37°C avant les mesures).

Préparation de l'échantillon :

Identique à l'exemple 14.

Procédure de dosage :

Identique à l'exemple 17, si ce n'est que :

- Chaque réaction est déclenchée par l'ajout de 30 µl de réactif **S1**.
- Le milieu réactionnel est préparé extemporanément de la façon suivante :

| | **TEMOIN** | **ECHANTILLON** |
|---|---|---|
| **S2** | 30 µl | 30 µl |
| **Blanc échantillon (*)** | 40 µl | - |
| **ECHANTILLON** | - | 40 µl |
| **S3** | 900 µl | 900 µl |

(*) : Eau distillée ayant subi la même procédure d'extraction que le lysat érythrocytaire.

Expression des résultats :

L'activité SOD de l'échantillon est obtenue à partir du rapport $V_s/V_c$, dont la variation avec la concentration en SOD (exprimée en unités d'activité SOD) est donnée par l'équation suivante :

$$V_s/V_c = 1 + [SOD]/(0{,}073 \times [SOD] + 0{,}93).$$

Dans les conditions de ce dosage, une unité d'activité SOD correspond à un rapport $V_s/V_c$ égal à 2 et est appelée unité SOD-525 (car l'évolution de l'absorbance est suivie à 525 nm, comme dans l'exemple 17).

La figure 7 annexée représente la variation du rapport $V_s/V_c$ en fonction de la concentration en SOD exprimée en unités SOD-525/ml, dans cet exemple.

$$\text{Unités d'activité SOD}=[0{,}93x(1-V_s/Vc)]/[0{,}073x(V_s/V_c-1)-1]xD.$$

REFERENCES

1. FRIDOVICH I. (1986), Biological effects of the superoxide radical; Arch. Biochem. Biophys. 247, 1-11.

2. FRIDOVICH I. (1986a), Superoxide dismutases; Adv. Enzymol. 58, 61-97.

3. FRIDOVICH I. (1989), Superoxide dismutases; an adaptation to a paramagnetic gas; J. Biol. Chem. 264, 7761-7764.

4. KARLSSON K. et MARKLUND S.L. (1988), Extracellular superoxide dismutase in the vascular system of mammals; Biochem. J., 255, 223-228.

5. CZAPSKI G. et GOLDSTEIN S. (1988), The ubiquiness both of superoxide toxicity and of the protective role of superoxide dismutase; dans: Oxygen Radicals in Biology and Medecine, SIMIC M.G., TAYLOR K.A., WARD J.F. et VON SONNTAG C. Ed., Plenum Press, New-York et Londres, 43-46.

6. FRIDOVICH I. (1982b), Measuring the activity of superoxide dismutases: an embarrassment of riches; dans: Superoxide dismutase Vol-I, OBERLEY L.W. Ed., CRC Press, Boca Raton, Floride, 69-77.

7. FLOHE L. et OTTING F. (1984), Superoxide dismutase assays; Meth. Enzymol., 105, 93-104.

8. BANNISTER J.V. et CALABRESE (1987), Assays for superoxide dismutase; dans : Methods of Biochemical Analysis, Vol. 32, John Wiley & Sons,.279-311.

9. FLOHE L. BECKER R., BRIGELIUS R., LENGFELDER E. et OTTING F. (1989), Convenient assays for superoxide dismutase; dans: CRC Handbook; Free Radicals and Antioxidants in Biomedicine, Vol.III, MIQUEL J., WEBER H. et QUINTANILHA A. Eds., CRC Press, Boca Raton, bride, 287-293.

10. GOLDSTEIN S., MICHEL C., BORS W., SARAN M. et CZAPSKI G. (1988), A critical reevaluation of some assay methods for superoxide dismutase activity; Free Rad. Biol. Med., 4, 295-303.

11. MARKLUND S.L. (1976), Spectrophotometric study of spontaneous disproportionation of superoxide anion radical and sensitive direct assay for superoxide dismutase; J. Biol. Chem., 251, 7504-7507.

12. MARKLUND S.L. (1985), Quantitation of superoxide dismutase: Direct assay wich potassium superoxide; dans : CRC Handbook of Methods for Oxygen Radical Research, GREENWALD R.A. Ed., CRC Press, Boca Raton, Floride, 249-255.

13. MARTIN J.P., DAILEY M. et SUGARMAN E. (1987), Negative and positive assays of superoxide dismutase based on hematoxylin autoxidation; Arch. Biochem. Biophys., 255, 329-336.

14. MARTIN J.P. (1990), Assays for superoxide dismutase based on autoxidation of hematoxylin; Meth. Enzymol., 186, 220-227.

15. TRUCE, KREIDER et BRAND (1971), Org. React., 18, 99-215.

16. COMINS D.L., STROUD E.D. et HERRICK J.J. (1984), Regioselective alkylation of Grignard reagents to the 1-phenoxycarbonyl salts of alkyl nicotinates ; Heterocycles, 22(1), 151-157.

**Revendications**

**1.** Procédé de dosage de l'activité superoxyde dismutase (SOD) dans un milieu liquide, procédé qui utilise l'activation de l'autoxydation d'un réactif par l'activité SOD, caractérisé

- en ce que ledit réactif est un composé répondant à la formule générale I :

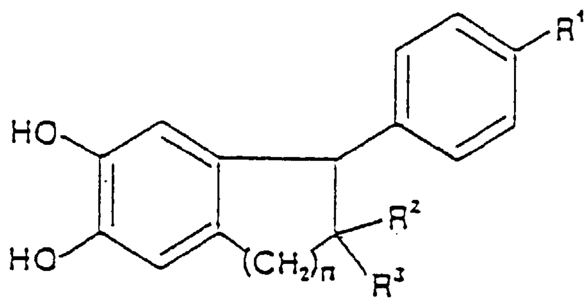

(I)

dans laquelle :

<u>soit</u> :

n = 1 ou 2,

$R^1$ = $-OR^4$ ou $-NR^5R^6$,

$R^2$ = hydrogène, $-OR^4$, alkyle (avec 1 à 6 atomes de carbone), ou bien $-CH_2-$ ou $-CH_2-CH_2-$, pour former un cycle par liaison avec le substituant phényle, en position méta par rapport à $R^1$ et

$R^3$ = hydrogène, alkyle (avec 1 à 6 atomes de carbone) ou $-OR^4$ (à condition que $R^2$ soit différent de $-OR^4$), avec

$R^4$ = hydrogène ou alkyle (avec 1 à 6 atomes de carbone),

$R^5$ = hydrogène, alkyle (avec 1 à 6 atomes de carbone), $-CH_2COOH$, $-C_6H_5COOH$ ou $-C_6H_5SO_3H$ et

$R^6$ = hydrogène, alkyle (avec 1 à 6 atomes de carbone) ou $-CH_2COOH$ ;

<u>soit</u> :

n = 1,

$R^1$ = $-OR^4$ ($R^4$ étant défini comme précédemment),

$R^2$ = $-CH_2-O-$, pour former un cycle par liaison de l'atome d'oxygène avec le substituant phényle, en position méta par rapport à $R^1$, et

$R^3$ = hydrogène ou $-OR^4$ ($R^4$ étant défini comme précédemment),

et

- en ce que ledit procédé repose sur une mesure spectrophotométrique de la vitesse d'autoxydation dudit composé de formule générale I, en l'absence et en présence de l'échantillon à doser.

**2.** Procédé de dosage de l'activité superoxyde dismutase (SOD) dans un milieu liquide contenant un ou plusieurs mercaptan(s), notamment un milieu biologique, procédé qui utilise l'activation de l'autoxydation d'un réactif par l'activité SOD, caractérisé

- en ce que ledit réactif est un composé répondant à la formule générale I définie à la revendication 1,
- en ce que le milieu contient en outre une quantité, capable de piéger totalement lesdits mercaptans par S-alkylation, d'au moins un composé répondant à la formule générale II :

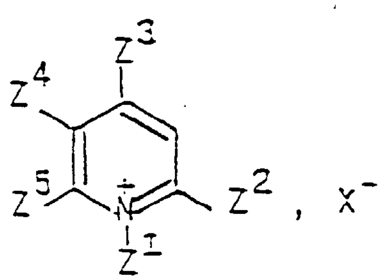

(II)

dans laquelle :

$Z^1$ = alkyle (avec 1 à 6 atomes de carbone), benzyle, p-nitrobenzyle, phényle, o,p-dinitrophényle, $-CH_2-COOH$ ou $-CH_2-CH_2-COOH$;

$Z^4$ = hydrogène et

$Z^5$ = hydrogène ou alkyle (avec 1 à 6 atomes de carbone),

ou

$Z^4$ et $Z^5$ forment ensemble, avec les deux atomes de carbone intermédiaires, un cycle phényle;
X = halogénure, sulfonate, fluorasulfonate, phosphonate, tétrafluoroborate ou tosylate; et

soit

$Z^2$ = vinyle, et
$Z^3$ = hydrogène ou alkyle (avec 1 à 6 atomes de carbone);

soit

$Z^3$ = vinyle, et
$Z^2$ = hydrogène ou alkyle (avec 1 à 6 atomes de carbone),et

- en ce que ledit procédé repose sur une mesure spectrophotométrique de la vitesse d'autoxydation dudit composé de formule générale I, en l'absence et en présence de l'échantillon à doser.

**3.** Procédé selon la revendication 2, caractérisé en ce que, dans la formule générale II, X = trifluorométhylsulfonate.

**4.** Procédé de dosage de l'activité superoxyde dismutase (SOD) dans un échantillon liquide, caractérisé en ce qu'il comprend essentiellement les étapes consistant à :

1°) déterminer la vitesse maximale d'autoxydation Vs d'un composé de formule générale I en présence de l'échantillon, au moyen de l'évolution de l'absorbance en fonction du temps, à la longueur d'onde caractérisant l'apparition du produit d'autoxydation du composé de formule générale I utilisé, dans un milieu réactionnel tamponné à une valeur de pH de 8,0 à 9,0, en déclenchant la réaction par ajout d'un aliquot d'une solution-mère du composé de formule générale I ;
2°) déterminer, dans les mêmes conditions, la vitesse maximale d'autoxydation Vc du même composé en l'absence de l'échantillon ; et
3°) déterminer l'activité SOD de l'échantillon au moyen des vitesses Vs et Vc obtenues et d'une courbe d'étalonnage établie dans les mêmes conditions opératoires.

**5.** Procédé selon l'une quelconque des revendications 1 à 4, caractérisé en ce qu'il comprend l'ajout d'un dérivé du bore, notamment l'acide borique, pour moduler la vitesse d'autoxydation du composé de formule générale I.

**6.** Procédé selon la revendication 4 ou 5, caractérisé en ce qu'on combine à la mesure faite dans des conditions d'activation de l'autoxydation du composé de formule générale I, une mesure effectuée dans des conditions d'inhibition de cette autoxydation, dans une gamme de pH de 7,2 à 7,8.

**7.** Procédé selon l'une quelconque des revendications 1 à 6, caractérisé en ce que le composé de formule générale I utilisé est le 5,6,6a,11b-tétrahydro-3,9,10-trihydroxybenzo[c]fluorène.

**8.** Nécessaire ou kit pour la mise en oeuvre du procédé selon l'une quelconque des revendications 1 à 6, caractérisé en ce qu'il comprend essentiellement, en tant que réactif, un composé de formule générale I.

**9.** Nécessaire ou kit selon la revendication 8, caractérisé en ce qu'il comprend en outre un ou plusieurs composé(s) piégeur(s) de mercaptans de formule générale II.

**10.** Nécessaire ou kit selon la revendication 9, caractérisé en ce qu'il comprend essentiellement :

- un composé de formule générale I en solution acide ou en poudre,
- un ou plusieurs composé(s) piégeur(s) de mercaptans de formule générale II, en solution ou en poudre, et
- un tampon à base de AMPD, tamponnant dans la gamme de pH de 8,0 à 9,0.

**11.** Composés de formule générale I dans laquelle $R^1$ à $R^3$ sont définis comme à la revendication 1, étant entendu

que lorsque n = 1 et $R^2$ = $-CH_2-O-$, $R^1$ en $R^3$ ne peuvent représenter simultanément $-OR^4$, avec $R^4$ = hydrogène.

**12.** Composés de formule générale I :

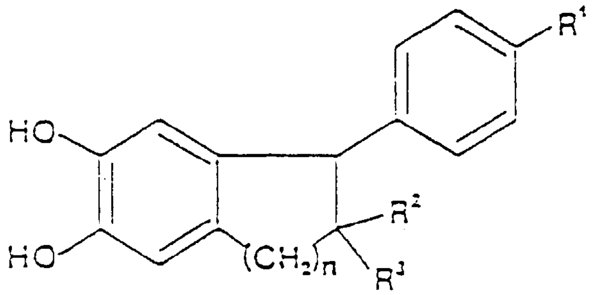

(I)

dans laquelle :

n = ou 2,
$R^1$ = $-OR^4$ ou $-NR^5R^6$,
$R^2$ = hydrogène, $-OR^4$, alkyle (avec 1 à 6 atomes de carbone), ou bien $-CH_2-$ cu $-CH_2-CH_2-$, pour former un cycle par liaison avec le substituant phényle, en position méta par rapport à $R^1$ et
$R^3$ = hydrogène, alkyle (avec 1 à 6 atomes de carbone) ou $-OR^4$ (à condition que $R^2$ soit différent de $-OR^4$), avec
$R^4$ = hydrogène ou alkyle (avec 1 à 6 atomes de carbone),
$R^5$ = hydrogène, alkyle (avec 1 à 6 atomes de carbone), $-CH_2COOH$, $-C_6H_5COOH$ ou $-C_6H_5SO_3H$ et
$R^6$ = hydrogène, alkyle (avec 1 à 6 atomes de carbone) ou $-CH_2COOH$.

**13.** Préparation du composé de formule générale I dans laquelle n = 1, $R^1$ = OH, $R^2$ = $-CH_2-O-$ et $R^3$ = hydrogène, ou brazilane, caractérisé en ce qu'il comprend essentiellement les étapes consistant à :

- protéger la braziline sur ses hydroxyles phénoliques,
- transformer l'hydroxyle alcoolique en un groupe permettant son élimination, et
- réduire et déprotéger le produit résultant.

**Patentansprüche**

**1.** Verfahren zur Messung der Superoxid-Dismutase (SOD)-Aktivität in einem flüssigen Medium, bei welchem die Aktivierung der Autooxidation eines Reagens durch durch die SOD-Aktivität verwendet wird,

dadurch gekennzeichnet, daß das Reagens eine Verbindung der allgemeinen Formel (I) ist:

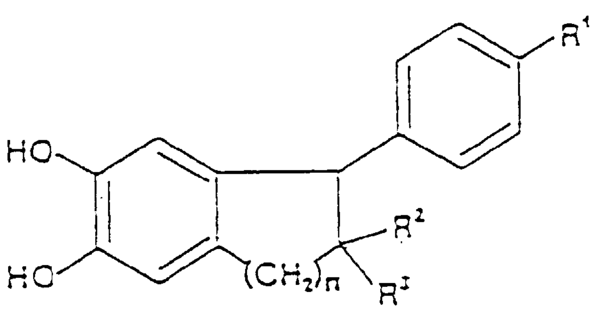

(I),

worin
entweder: n = 1 oder 2; $R^1$ = $-OR^4$ oder $-NR^5R^6$; $R^2$ = Wasserstoff, $-OR^4$, Alkyl (mit 1 bis 6 Kohlenstoffatomen) oder auch $-CH_2-$ oder $-CH_2-CH_2-$, zur Bildung eines Rings durch den Zusammenschluß mit dem Phenyl-Substituenten in meta-Stellung in bezug auf $R^1$; $R^3$ = Wasserstoff, Alkyl (mit 1 bis 6 Kohlenstoffatomen) oder $-OR^4$ (mit der Maßgabe, daß $R^2$ von $-OR^4$ verschieden ist); $R^4$ = Wasserstoff oder Alkyl (mit 1 bis 6 Kohlenstoffatomen); $R^5$ = Wasserstoff, Alkyl (mit 1 bis 6 Kohlenstoffatomen), $-CH_2COOH$, $-C_6H_5COOH$ oder $-C_6H_5SO_3H$; und $R^6$ = Wasserstoff, Alkyl (mit 1 bis 6 Kohlenstoffatomen) oder $-CH_2COOH$;

oder: n = 1; $R^1$ = $-OR^4$ (wobei $R^4$ wie vorstehend definiert ist); $R^2$ = $-CH_2-O-$, zur Bildung eines Rings durch den Zusammenschluß des Sauerstoffatoms mit dem Phenyl-Substituenten in meta-Stellung in bezug auf $R^1$; $R^3$ = Wasserstoff oder $-OR^4$ (wobei $R^4$ wie vorstehend definiert ist);
und daß das Verfahren auf einer spektrophotometrischen Messung der Autooxidationsgeschwindigkeit der Verbindung der allgemeinen Formel (I) in Abwesenheit und in Anwesenheit einer zu messenden Probe basiert.

**2.** Verfahren zur Messung der Superoxid-Dismutase (SOD)-Aktivität in einem flüssigen Medium, das ein oder mehrere Mercaptane enthält, insbesondere einem biologischen Medium, bei welchem Verfahren die Aktivierung der Autooxidation eines Reagens durch durch die SOD-Aktivität verwendet wird,

dadurch gekennzeichnet, daß das Reagens eine Verbindung der allgemeinen Formel (I), wie in Anspruch 1 definiert, ist,
daß das Medium außerdem eine zum vollständigen Abfangen der Mercaptane durch S-Alkylierung geeignete Menge zumindest einer Verbindung der allgemeinen Formel (II) enthält:

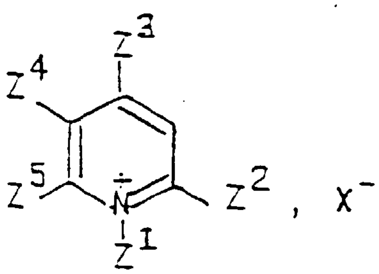

(II),

worin $Z^1$ = Alkyl (mit 1 bis 6 Kohlenstoffatomen), Benzyl, p-Nitrobenzyl, Phenyl, o,p-Dinitrophenyl, $-CH_2COOH$ oder $-CH_2-CH_2-COOH$; $Z^4$ = Wasserstoff, und $Z^5$ = Wasserstoff oder Alkyl (mit 1 bis 6 Kohlenstoffatomen), oder $Z^4$ und $Z^5$ zusammen mit den beiden dazwischenliegenden Kohlenstoffatomen einen Phenylring bilden; X = Halogenid, Sulfonat, Fluorsulfonat, Phosphonat, Tetrafluorborat oder Tosylat; und entweder $Z^2$ = Vinyl, und $Z^3$ = Wasserstoff oder Alkyl (mit 1 bis 6 Kohlenstoffatomen), oder $Z^3$ = Vinyl, und $Z^2$ = Wasserstoff oder Alkyl (mit 1 bis 6 Kohlenstoffatomen);
und daß das Verfahren auf einer spektrophotometrischen Messung der Autooxidationsgeschwindigkeit der Verbindung der allgemeinen Formel (I) in Abwesenheit und in Anwesenheit einer zu messenden Probe basiert.

**3.** Verfahren nach Anspruch 2, dadurch gekennzeichnet, daß in der allgemeinen Formel (II) X = Trifluormethylsulfonat.

**4.** Verfahren zur Messung der Superoxid-Dismutase (SOD)-Aktivität in einer flüssigen Probe, dadurch gekennzeichnet, daß es im wesentlichen die Schritte umfaßt, die bestehen aus:

1) Bestimmen der maximalen Autooxidationsgeschwindigkeit Vs einer Verbindung der allgemeinen Formel (I) in Anwesenheit einer Probe mittels der Entwicklung des Absorptionsvermögens als Funktion der Zeit bei einer Wellenlänge, die das Auftreten des Autooxidationsprodukts der verwendeten Verbindung der allgemeinen Formel (I) charakterisiert, in einem Reaktionsmedium, das auf einen pH-Wert von 8,0 bis 9,0 gepuffert wird, wobei die Reaktion durch den Zusatz einer aliquoten Menge einer Mutterlauge der Verbindung der allgemeinen Formel (I) ausgelöst wird;
2) Bestimmen der maximalen Autooxidationsgeschwindigkeit Vc derselben Verbindung unter denselben Bedingungen in Abwesenheit der Probe; und
3) Bestimmen der SOD-Aktivität der Probe mittels der erhaltenen Geschwindigkeiten Vs und Vc und einer unter denselben Betriebsbedingungen festgelegten Eichkurve.

**5.** Verfahren nach einem der Ansprüche 1 bis 4, dadurch gekennzeichnet, daß es den Zusatz eines Bor-Derivats, insbesondere von Borsäure, zur Modulation der Autooxidationsgeschwindigkeit der Verbindung der allgemeinen Formel (I) umfaßt.

**6.** Verfahren nach Anspruch 4 oder 5, dadurch gekennzeichnet, daß mit der unter den Aktivierungsbedingungen der Autooxidation der Verbindung der allgemeinen Formel (I) durchgeführten Messung eine unter Inhibierungsbedingungen dieser Autooxidation vorgenommene Messung in einem pH-Bereich von 7,2 bis 7,8 kombiniert wird.

**7.** Verfahren nach einem der Ansprüche 1 bis 6, dadurch gekennzeichnet, daß die verwendete Verbindung der allgemeinen Formel (I) 5,6,6a,11b-Tetrahydro-3,9,10-trihydroxybenzo[c]fluoren ist.

**8.** Testsatz oder Kit zur Durchführung eines Verfahrens nach einem der Ansprüche 1 bis 6, dadurch gekennzeichnet, daß er im wesentlichen als Reagens eine Verbindung der allgemeinen Formel (I) umfaßt.

**9.** Testsatz oder Kit nach Anspruch 8, dadurch gekennzeichnet, daß er außerdem eine oder mehrere Mercaptan-Fänger-Verbindungen der allgemeinen Formel (II) umfaßt.

**10.** Testsatz oder Kit nach Anspruch 9, dadurch gekennzeichnet, daß er im wesentlichen umfaßt:

eine Verbindung der allgemeinen Formel (I) in sauer Lösung oder als Pulver,
eine oder mehrere Mercaptan-Fänger-Verbindungen der allgemeinen Formel (II) in Lösung oder als Pulver, und
einen Puffer auf der Basis von AMPD, der in einem pH-Bereich von 8,0 bis 9,0 puffert.

**11.** Verbindung der allgemeinen Formel (I), worin $R^1$ und $R^3$ wie in Anspruch 1 definiert sind, mit der Maßgabe, daß, wenn n = 1, und $R^2$ = $-CH_2-O-$, $R^1$ und $R^3$ nicht gleichzeitig $-OR^4$ sein können, wobei $R^4$ = Wasserstoff.

**12.** Verbindung der allgemeinen Formel (I):

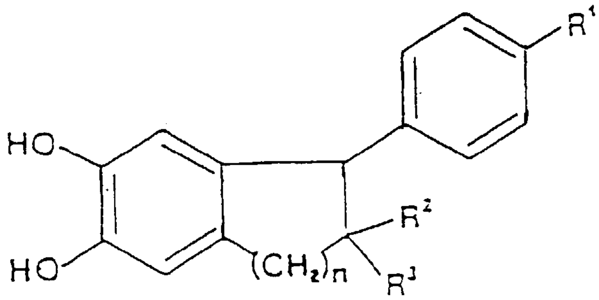

(I),

worin n = 1 oder 2; $R^1$ = $-OR^4$ oder $-NR^5R^6$; $R^2$ = Wasserstoff, $-OR^4$, Alkyl (mit 1 bis 6 Kohlenstoffatomen) oder auch $-CH_2-$ oder $-CH_2-CH_2-$, zur Bildung eines Rings durch den Zusammenschluß mit dem Phenyl-Substituenten in meta-Stellung in bezug auf $R^1$; $R^3$ = Wasserstoff, Alkyl (mit 1 bis 6 Kohlenstoffatomen) oder $-OR^4$ (mit der Maßgabe, daß $R^2$ von $-OR^4$ verschieden ist); $R^4$ = Wasserstoff oder Alkyl (mit 1 bis 6 Kohlenstoffatomen); $R^5$ = Wasserstoff oder Alkyl (mit 1 bis 6 Kohlenstoffatomen), $-CH_2COOH$, $-C_6H_5COOH$ oder $-C_6H_5SO_3H$; und $R^6$ = Wasserstoff, Alkyl (mit 1 bis 6 Kohlenstoffatomen) oder $-CH_2COOH$.

**13.** Verfahren zur Herstellung einer Verbindung der allgemeinen Formel (I), worin n = 1, $R^1$ = OH, $R^2$ = $-CH_2-O-$, und $R^3$ = Wasserstoff oder Brazilan, dadurch gekennzeichnet, daß es im wesentlichen die Schritte umfaßt, die bestehen aus:

Schützen des Brazilins an seinen phenolischen Hydroxylen,
Transformieren des alkoholischen Hydroxyls in eine Gruppe, die seine Eliminierung ermöglicht, und
Reduzieren und Entschützen des erhaltenen Produkts.

**Claims**

**1.** Process for the assay of superoxide dismutase (SOD) activity in a liquid medium, which process utilized activation of the autoxidation of a reagent by SOD activity, characterized

in that the said reagent is a compound conforming to the general formula (I):

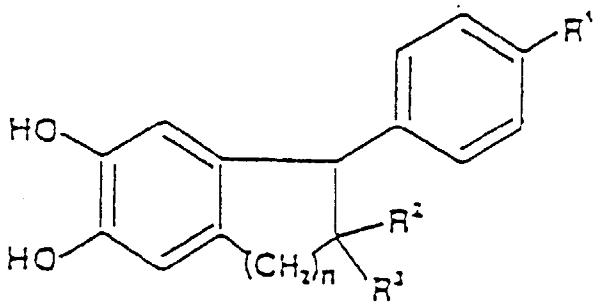

(I)

in which:
<u>either</u>:

n = 1 or 2,
$R^1$ = $-OR^4$ or $-NR^5R^6$,
$R^2$ = hydrogen, $-OR_4$, alkyl (with 1 to 6 carbon atoms), or else $-CH_2-$ or $-CH_2-CH_2-$, to form a ring by linking with the phenyl substituent, in the meta position in relation to $R^1$, and
$R^3$ = hydrogen, alkyl (with 1 to 6 carbon atoms) or $-OR^4$ (with the proviso that $R^2$ is different from $-OR^4$), where
$R^4$ = hydrogen or alkyl (with 1 to 6 carbon atoms),
$R^5$ = hydrogen, alkyl (with 1 to 6 carbon atoms), $-CH_2COOH$, $-C_6H_5COOH$ or $-C_6H_5SO_3H$, and
$R^6$ = hydrogen, alkyl (with 1 to 6 carbon atoms) or $-CH_2COOH$;

or:

n = 1,
$R^1$ = $-OR^4$ ($R^4$ being defined as above),
$R^2$ = $-CH_2-O-$, to form a ring by linkage of the oxygen atom with the phenyl substituent, in the meta position in relation to $R^1$, and
$R^3$ = hydrogen or $-OR^4$ ($R^4$ being defined as above), and

- in that said process resides in a spectrophotometric measurement of the autoxidation rate of said compound of general formula I in the absence and the presence of the sample to be assayed.

**2.** Process for the assay of superoxide dismutase (SOD) activity in a liquid medium containing one or more mercaptan (s), especially a biological medium, which process utilizes activation of the autoxidation of a reagent by SOD activity, characterized

- in that the said reagent is a compound conforming to the general formula I defined in claim 1,
- in that the medium additionally contains a quantity, which is capable of totally scavenging the said mercaptans by S-alkylation, of at least one compound conforming to the general formula II:

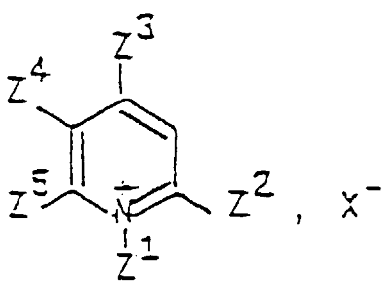

(II)

in which:

$Z^1$ = alkyl (with 1 to 6 carbon atoms), benzyl, p-nitrobenzyl, phenyl, o,p-dinitrophenyl, $-CH_2-COOH$ or $-CH_2-CH_2-COOH$;

$Z^4$ = hydrogen and
$Z^5$ = hydrogen or alkyl (with 1 to 6 carbon atoms), or
$Z^4$ and $Z^5$ form, together with the two intermediate carbon atoms, a phenyl ring;
X = halide, sulphonate, fluorosulphonate, phosphonate, tetrafluoroborate or tosylate; and

either

$Z^2$ = vinyl, and
$Z^3$ = hydrogen or alkyl (with 1 to 6 carbon atoms),

or

$Z^3$ = vinyl, and
$Z^2$ = hydrogen or alkyl (with 1 to 6 carbon atoms), and

- in that said process resides in a spectrophotometric measurement of the autoxidation rate of said compound of general formula I in the absence and the presence of the sample to be assayed.

**3.** Process according to claim 2, characterized in that in general formula II, X = trifluoromethylsulphonate.

**4.** Process for the assay of superoxide dismutase (SOD) activity in a liquid sample, characterized in that it essentially comprises the steps consisting in:

1) determining the maximum rate of autoxidation Vs of a compound of general formula I in the presence of the sample, by means of the change in the absorbance as a function of time, at the wavelength which characterizes the appearance of the autoxidation product of the compound of general formula I used, in a reaction medium which is buffered to a pH value of from 8.0 to 9.0, triggering the reaction by the addition of an aliquot of a stock solution of the compound of general formula I;
2) determining, under the same conditions, the maximum autoxidation rate $V_c$ of the same compound in the absence of the sample; and
3) determining the SOD activity of the sample by means of the rates $V_s$ and $V_c$ obtained and of a calibration curve established under the same operating conditions.

**5.** Process according to any one of claims 1 to 4, characterized in that it comprises the addition of a boron derivative, especially boric acid, to modulate the autoxidation rate of the compound of general formula I.

**6.** Process according to claim 4 or 5, characterized in that the measure carried out under the conditions of activation of the autoxidation of the compound of general formula I is combined with a measure carried out under conditions in which this autoxidation is inhibited, in a pH range from 7.2 to 7.8.

**7.** Process according to any one of claims 1 to 6, characterized in that the compound of general formula I used is 5,6,6a,11b-tetrahydro-3,9,10-trihydroxybenzo[c] fluorene.

**8.** Kit for the implementation of the process according to any one of claims 1 to 6, characterized in that it essentially comprises, as reagent, a compound of general formula I.

**9.** Kit according to claim 8, characterized in that it also comprises one or more mercaptan-scavenging compound(s) of general formula II.

**10.** Kit according to claim 9, characterized in that it essentially comprises:

- a compound of general formula I in acidic solution or in a powder,
- one or more mercaptan-scavenging compound(s) of general formula II, in solution or as a powder, and
- a buffer based on AMPD, which buffers in the pH range from 8.0 to 9.0.

**11.** Compounds of general formula I in which $R^1$ to $R^3$ are as defined in claim 1, with the proviso that, when n = 1 and $R^2$ = $-CH_2-O-$, $R^1$ and $R^3$ may not simultaneously represent $-OR^4$ where $R^4$ = hydrogen.

**12.** Compounds of general formula I:

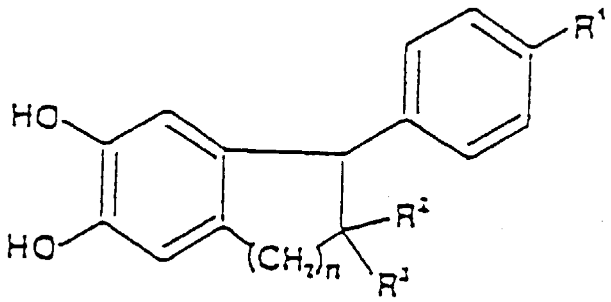

(I)

in which:

n = 1 or 2,
$R^1$ = $-OR^4$ or $-NR^5R^6$
$R^2$ = hydrogen, $-OR_4$, alkyl (with 1 to 6 carbon atoms), or else $-CH_2-$ or $-CH_2-CH_2$, to form a ring by linking with the phenyl substituent, in the meta position in relation to $R^1$, and
$R^3$ = hydrogen, alkyl (with 1 to 6 carbon atoms) or $-OR^4$ (with the proviso that $R^2$ is different from $-OR^4$), where
$R^4$ = hydrogen or alkyl (with 1 to 6 carbon atoms),
$R^5$ = hydrogen, alkyl (with 1 to 6 carbon atoms), $-CH_2COOH$, $-C_6H_5COOH$ or $-C_6H_5SO_3H$, and
$R^6$ = hydrogen, alkyl (with 1 to 6 carbon atoms) or $-CH_2COOH$.

**13.** Preparation of the compound of general formula I in which n = 1, $R^1$ = OH, $R^2$ = $-CH_2-O-$ and $R^3$ = hydrogen, or brazilane, characterized in that it essentially comprises the steps consisting in:

- protecting brazilin on its phenolic hydroxyls,
- transforming the alcoholic hydroxyl into a group which enables its removal, and
- reducing and deprotecting the resulting product.

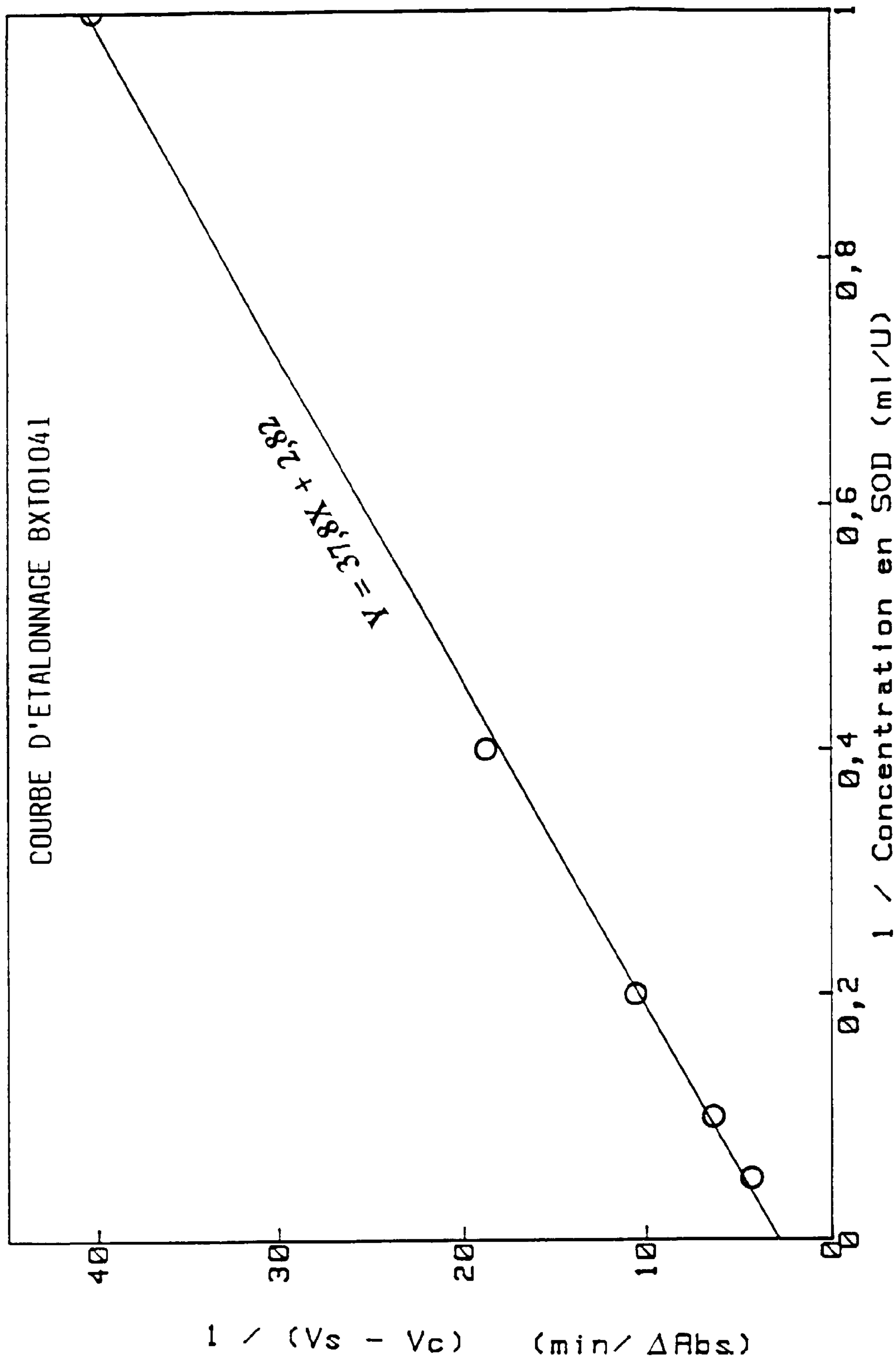
COURBE D'ETALONNAGE BXT01041
Y = 37,8X + 2,82
1 / Concentration en SOD (ml/U)
0
0,2
0,4
0,6
0,8
1
1 / (Vs - Vc) (min/ ΔAbs)
0
10
20
30
40

**Fig. 1**

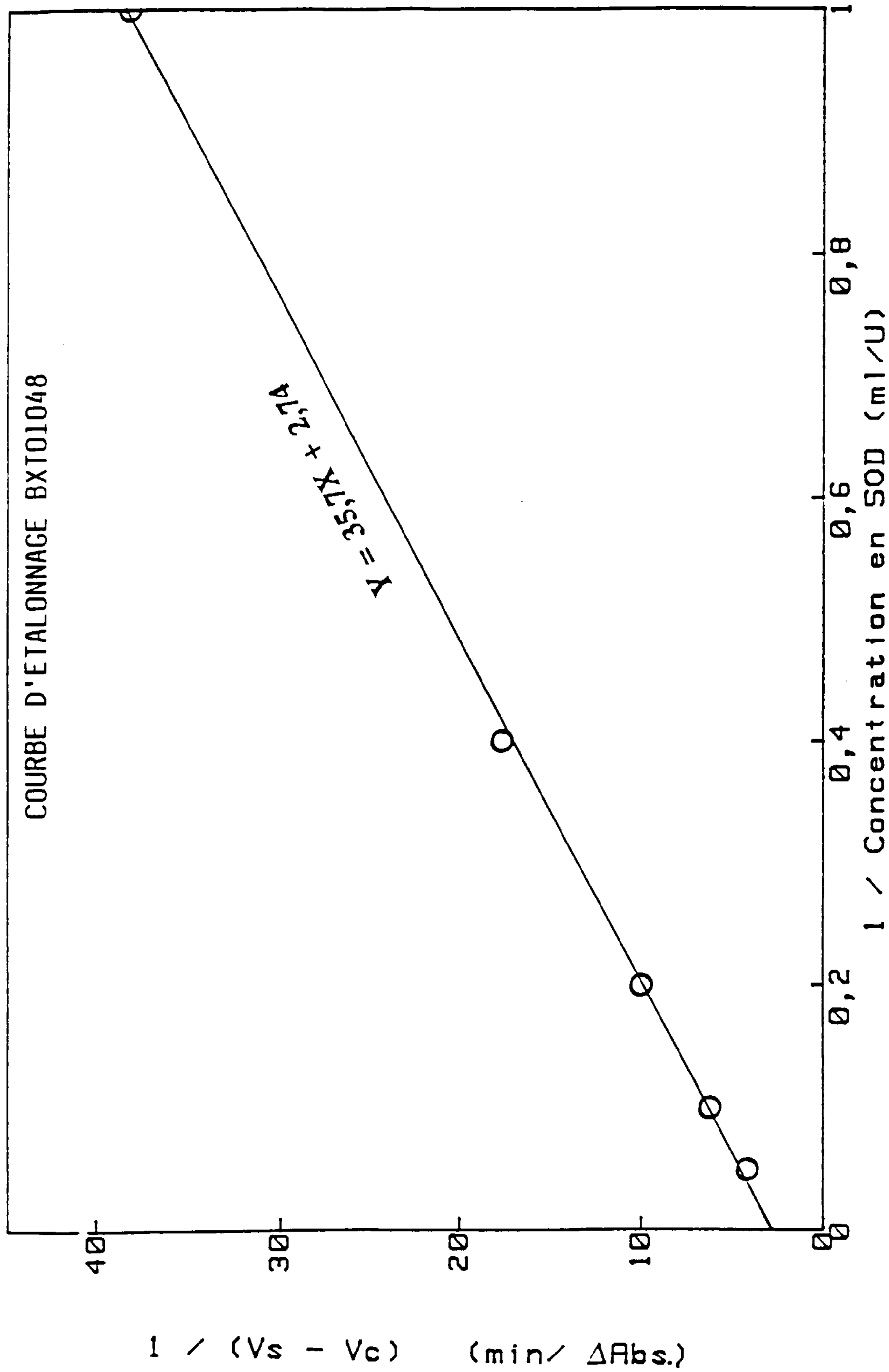
COURBE D'ETALONNAGE BXT01048
Y = 35,77X + 2,74
1 / Concentration en SOD (ml/U)
0
0,2
0,4
0,6
0,8
1
0
10
20
30
40
1 / (Vs - Vc) (min/ ΔAbs.)

Fig. 2

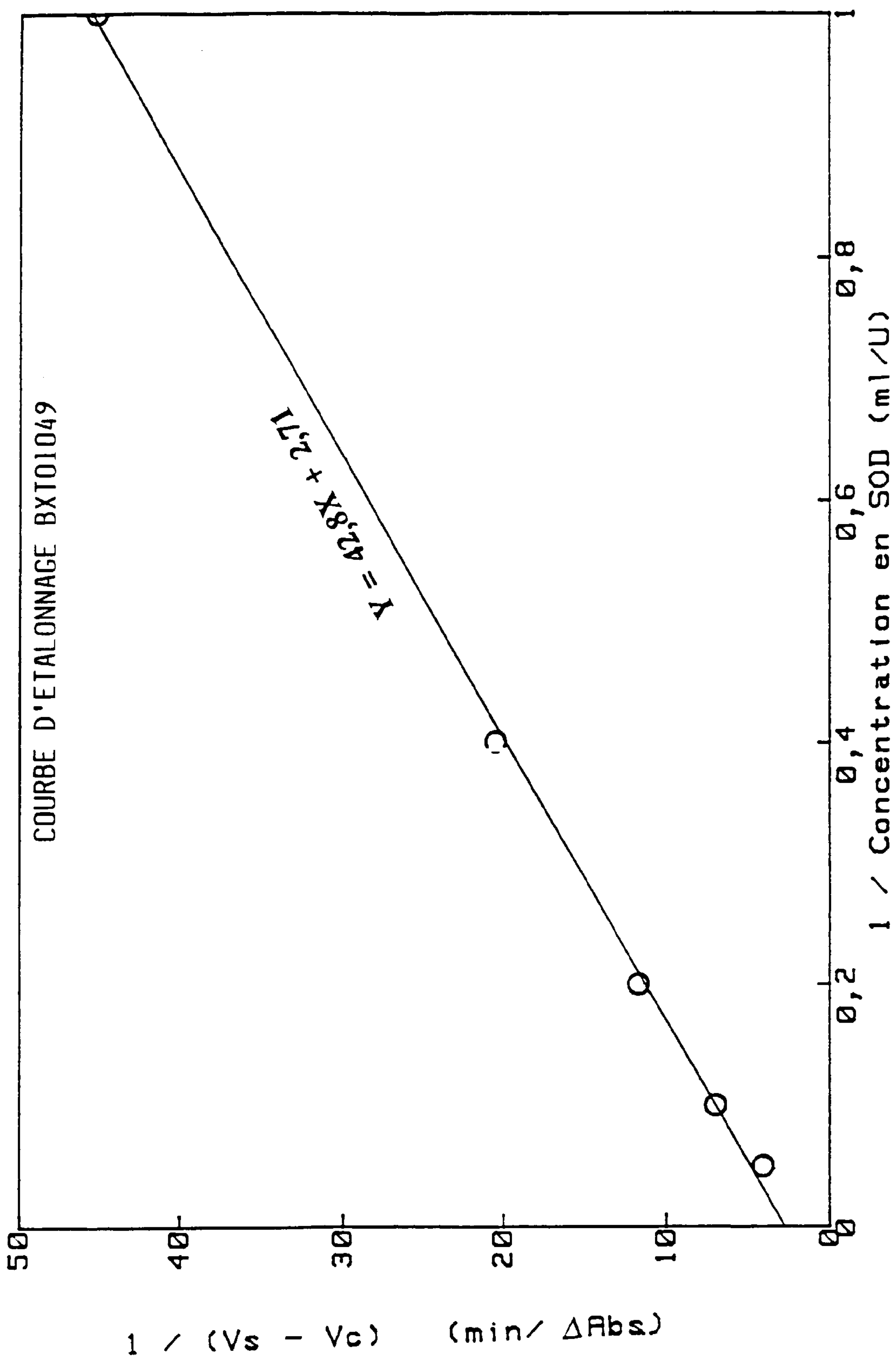
COURBE D'ETALONNAGE BXT01049
Y = 42,8X + 2,71
1 / Concentration en SOD (ml/U)
0
0,2
0,4
0,6
0,8
1
1 / (Vs - Vc) (min/ ΔAbs)
0
10
20
30
40
50

Fig. 3

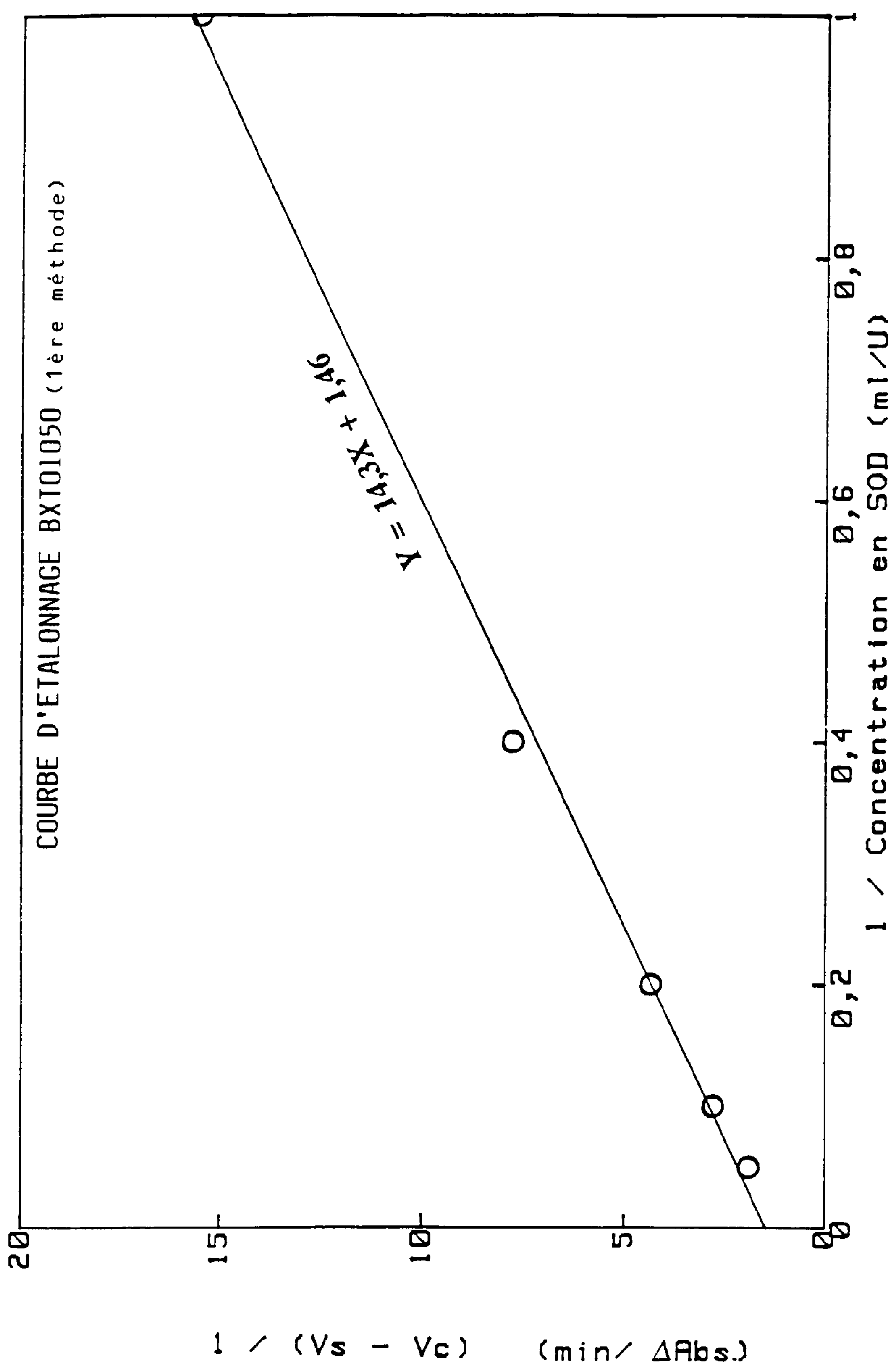
COURBE D'ETALONNAGE BXT01050 (1ère méthode)
Y = 14,3X + 1,46
1 / Concentration en SOD (ml/U)
0 0,2 0,4 0,6 0,8 1
1 / (Vs - Vc) (min/ ΔAbs.)
0 5 10 15 20

Fig. 4

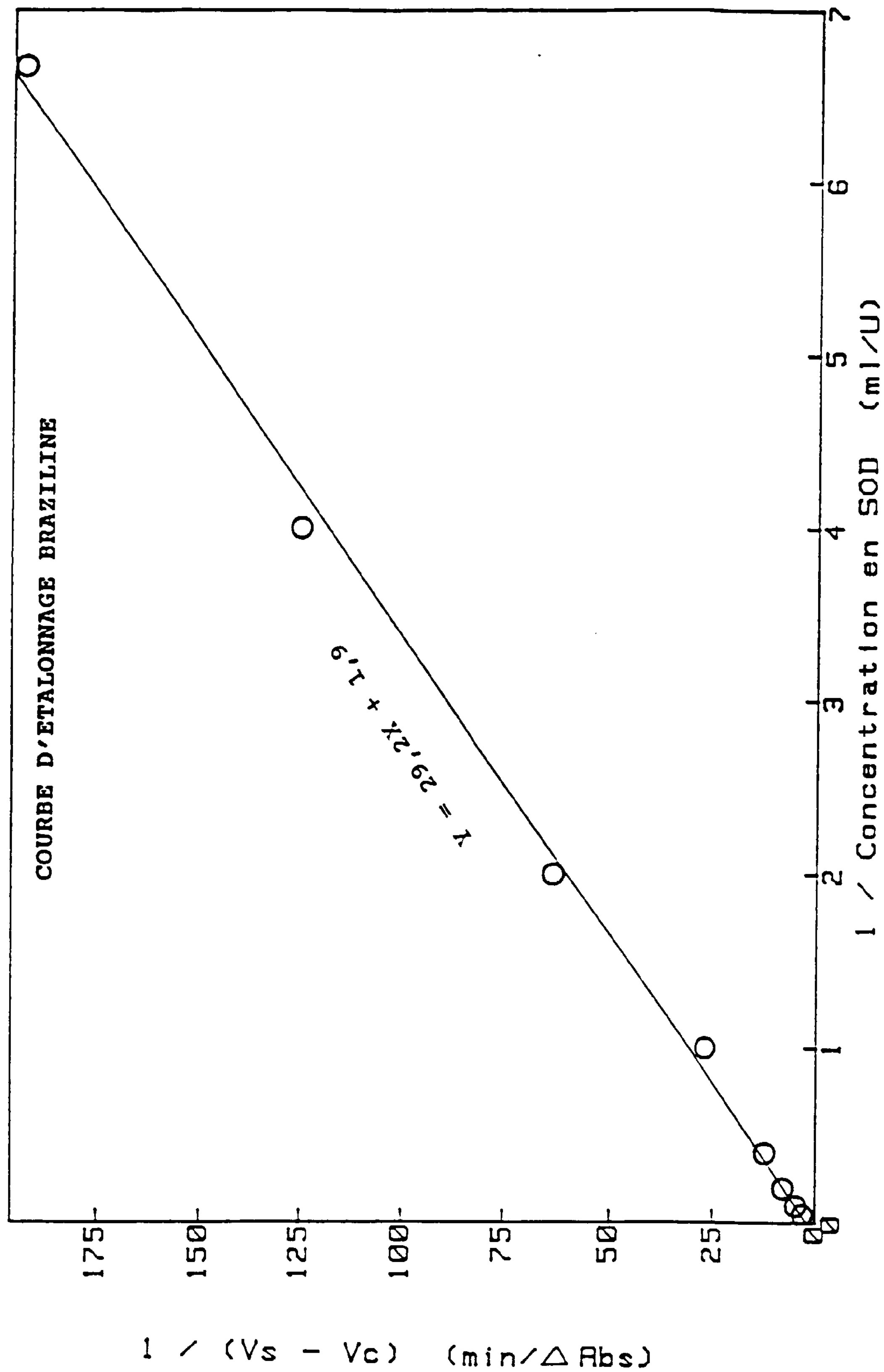
COURBE D'ETALONNAGE BRAZILINE
Y = 29,2X + 1,9
1 / Concentration en SOD (ml/U)
0
1
2
3
4
5
6
7
1 / (Vs - Vc) (min/Δ Abs)
0
25
50
75
100
125
150
175

Fig. 5

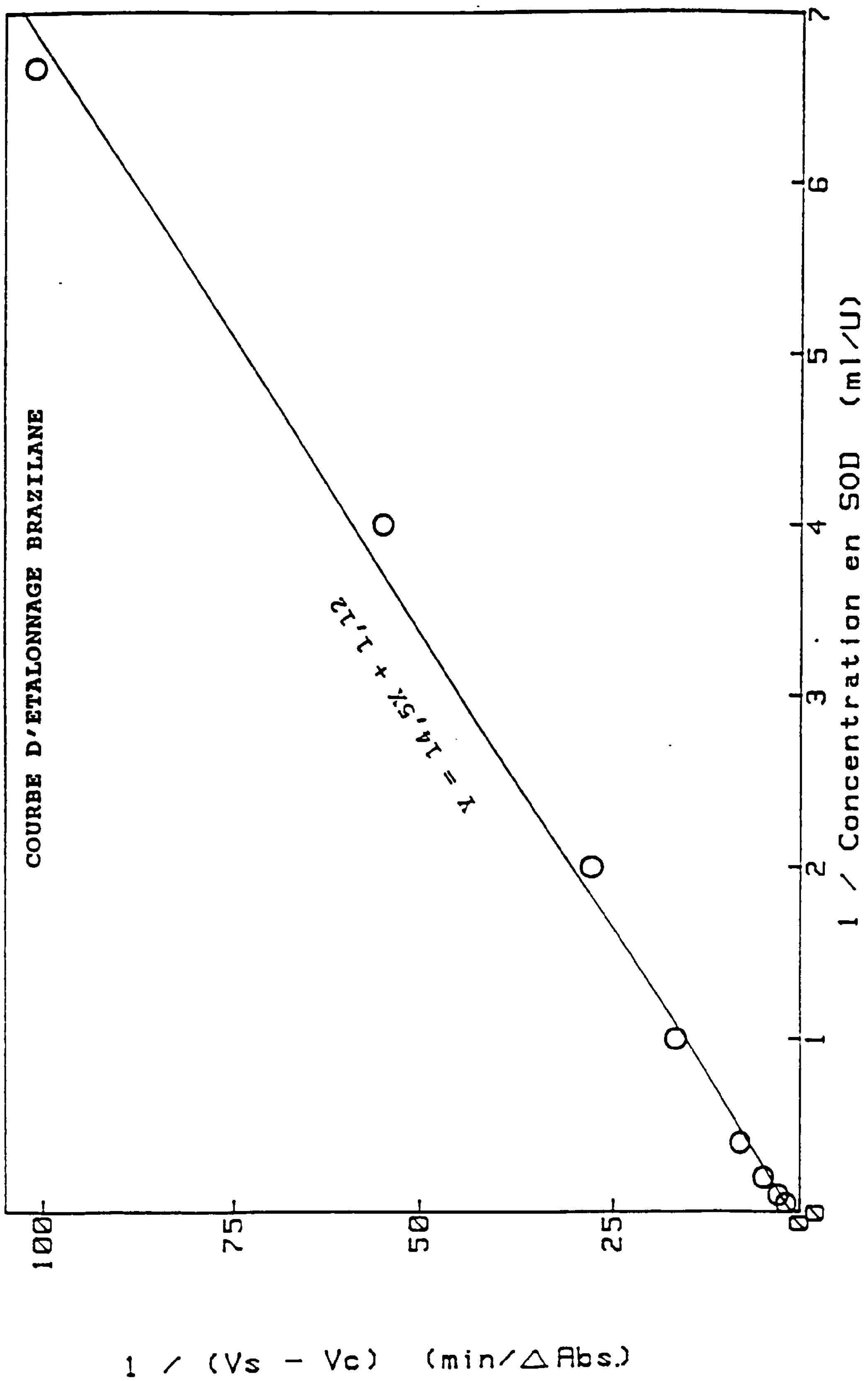
COURBE D'ETALONNAGE BRAZILANE
Y = 14,5X + 1,12
0
1
2
3
4
5
6
7
1 / Concentration en SOD (ml/U)
0
25
50
75
100
1 / (Vs - Vc) (min/ΔAbs.)

**Fig. 6**

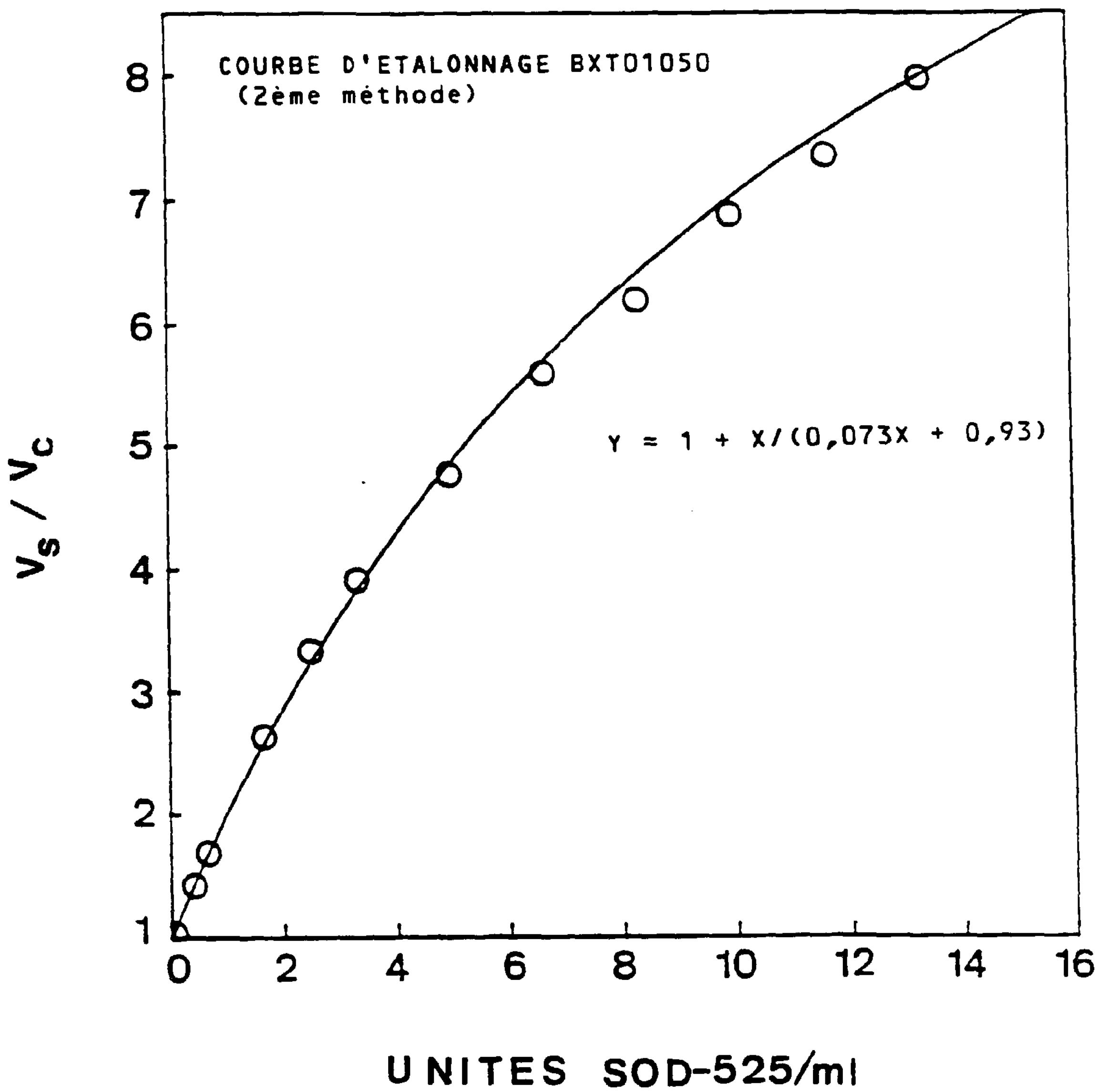
COURBE D'ETALONNAGE BXT01050
(2ème méthode)
Y = 1 + X/(0,073X + 0,93)
8
7
6
5
4
3
2
1
$V_s / V_c$
0
2
4
6
8
10
12
14
16
UNITES SOD-525/ml

Fig. 7